# EUROPEAN PATENT SPECIFICATION

(11) **EP 1 344 772 B1**
(45) Date of publication and mention of the grant of the patent: **22.07.2009**
(21) Application number: 01270523.2
(22) Date of filing: 14.12.2001
(51) Int. Cl.: C07C 317/10, C07C 315/00, B01J 31/22, C07C 69/78, C07C 67/08, C07C 47/445, C07C 45/69, C07F 1/02, C07F 1/10, C07F 5/00, B01J 31/02

(54) **ARYLBIS (PERFLUOROALKYLSULFONYL) METHANE, METAL SALT OF THE SAME, AND PROCESSES FOR PRODUCING THESE**
ARYLBIS(PERFLUORALKYLSULFONYL)-METHAN, DESSEN METALLSALZ UND VERFAHREN ZU DEREN HERSTELLUNG
ARYLBIS (PERFLUOROALKYLSULFONYL) METHANE, SON SEL METALLIQUE ET LEURS PROCEDES DE PRODUCTION

(30) Priority: 15.12.2000 JP 2000381537; 15.12.2000 JP 2000381539; 18.09.2001 JP 2001283216; 18.09.2001 JP 2001283217
(43) Date of publication of application: 17.09.2003
(73) Proprietor: Japan Science and Technology Agency, Kawaguchi-shi Saitama (JP)
(72) Inventor: ISHIHARA, Kazuaki, Konan-shi, Aichi 483-8012 (JP); YAMAMOTO, Hisashi, Nagoya-shi, Aichi 466-0815 (JP)
(74) Representative: Tanner, James Percival
(86) International application number: PCT/JP2001/010978
(87) International publication number: WO 2002/048098

(56) References cited:
- EP-A- 1 375 532
- JP-A- 3 254 697
- US-A- 4 049 861
- US-A- 4 431 845
- PATENT ABSTRACTS OF JAPAN vol. 1996, no. 01, 31 January 1996 (1996-01-31) & JP 07 246338 A (CENTRAL GLASS CO LTD), 26 September 1995 (1995-09-26)
- ISHIHARA, K. ET AL.: "Scandium Trifluoromethanesulfonate as an extremely active lewis acid catalyst in acylation of alcohols with acid anhydrides and mixed anhydrides" J.ORG.CHEM., vol. 61, 1996, pages 4560-4567, XP002313300
- ISHIHARA, KAZUAKI ET AL: "Polystyrene-bound tetrafluorophenylbis(triflyl)methane as an organic-solvent-swellable and strong bronsted acid catalyst" ANGEWANDTE CHEMIE, vol. 113, no. 21, 5 November 2001 (2001-11-05), pages 4201-4203, XP002313301
- ZHU, SHIZHENG: 'Synthesis and reactions of phenyliodonium bis(perfluoroalkanesulfonyl) methides' HETEROAT. CHEM. vol. 5, no. 1, 1994, pages 9 - 18, XP002909424
- ZHU, SHIZHENG: 'A new synthetic route to arylbis(perfluoroalkanesulfonyl)-methanes; structures of tolyldiazonium bis(trifluoromethanesulfonyl)methide and 4-nitrophenylhydrazonobis(trifluoromethanes ulfonyl)methane' J. FLUORINE CHEM. vol. 64, no. 1-2, 1993, pages 47 - 60, XP002051636
- LISTON, DAVID J. ET AL.: 'Observations on silver salt metathesis reactions with very weakly coordinating anions' J. AM. CHEM. SOC. vol. 111, no. 17, 1989, pages 6643 - 6648, XP002909425

## Description

### Technical Field

The present invention relates to a method for producing arylbis(perfluoroalkylsulfonyl)methane such as arylbis(triflyl)methane and the like, by using sodium trifluoromethane sulfinate and trifluoromethane sulfonic acid anhydride as an electrophilic reactant used as a triflyl source, and a novel arylbis(perfluoroalkylsulfonyl)methane such as pentafluorophenylbis(triflyl)methane, {4-(pentafluorophenyl)-2,3,5,6-tetrafluorophenyl}bis(trifly 1)methane and the like obtained from said method.

Moreover, the present invention relates to a metallic salt of arylbis(perfluoroalkylsulfonyl)methane, that is, a metallic arylbis(perfluoroalkylsulfonyl)methide, a method for producing said compound, catalysts which comprises said compound as an active ingredient and their use as Lewis acid catalysts, and a method for synthesizing organic compounds by using said catalysts.

### Background Art

Trifluoromethane sulfonyl (-SO₂CF₃; triflyl, Tf) group is known as one of the strongest electron-accepting group, which has an action to increase the protonic acidity of its a position (J. Am. Chem. Soc. 96, 2275, 1974; Synthesis, 691, 1997; J. Fluorine Chem. 66, 301, 1994). For example, bis(triflyl)methane (CH₂Tf₂; pKₐ(H₂O) = -1) (J. Am. Chem. Soc. 106, 1510, 1984) and phenylbis(triflyl)methane (PhCHTf₂; pKₐ(MeCN) = 7.83) (J. Org. Chem. 63, 7868, 1998) are strong acids that do not have the ability to oxidize. The inherent acidity ΔG_{acid} (in gas condition) estimated by Koppel et al. is as follows (J. Am. Chem. Soc. 116, 3047, 1994): MeSO₃H (315.0) < CH₂Tf₂ (310.5) < PhCHTf₂(310.3) < TfOH (299.5) < KHTf₂ (291.8) < CHTf₃ (289.0). These volatile crystalline solids are known to serve as a reactant when preparing a cationic organometallic dihydrido by protonating an organometallic hydride (J. Am. Chem. Soc. 106, 1510, 1984; J. Chem. Soc. , Chem. Commun. 1675, 1987; Inorg. Chem. 27, 1593, 1988; Inorg. Chem. 27, 2473, 1988; Organometallics 9, 1290, 1990). Based on these facts, it is expected that the steric and electronic effects of the aromatic group in the arylbis(triflyl)methane such as phenylbis(triflyl)methane and the like mentioned above, have a great effect on its Broensted acidity and the property of their organometallic complex.

Heretofore, two methods have been known as a method for synthesizing the phenylbis(triflyl)methane mentioned above (J. Org. Chem. 38, 3358, 1973; Heteroatom Chem. 5, 9, 1994; J. Fluorine Chem. 64, 47, 1993; J. Fluorine Chem. 106, 139, 2000). One of the methods is a method wherein benzyl magnesium chloride is reacted with triflyl fluoride to synthesize phenylbis(triflyl)methane (40% yield) (J. Org. Chem. 38, 3358, 1973), and the other method is a method wherein light response between iodobenzene bis(triflylmethide) and benzene is conducted (61% yield) (Heteroatom Chem. 5, 9, 1994). The former requires a triflyl fluoride gas (bp = -21° C) which is difficult to obtain, as a triflyl source, and the latter requires an excessive amount of benzene, a reactant, as a solvent. Moreover, in the case of the latter, arylbis(triflyl)methane is not formed when light response is conducted with allene, which has an electron-accepting group such as fluorobenzene.

Meanwhile, a method for synthesizing benzyl triflone has been reported by Hendrickson et al. (J. Am. Chem. Soc. 96, 2275, 1974; Synthesis, 691, 1997; J. Fluorine Chem. 66, 301, 1994). However, there was a problem that arylmethyl triflone could not be synthesized at a high yield when the aromatic group is an electron-accepting group and is inactivated (Synthesis, 691, 1997).

In addition, Lewis acid catalyst is known to be the most widely used catalyst in the aspect of organic synthesis. This Lewis acid catalyst associates with a specific functional group of an organic compound, forms a complex, and can be made to conduct a particular response only. The one that accepts an electron pair from which it reacts with is referred to as Lewis acid. Organic compounds generally have a functional group, and the functional group is usually a Lewis base, which attracts mutually with Lewis acid. The Lewis acid catalyst designed in this manner forms a complex with the functional group of the organic compound, and leads directly to the desired reaction. Due to this point, Lewis acid catalyst is also compared to an artificial enzyme. However, the reactivity and selectivity of the conventional Lewis acid catalyst was not so high compared to when enzyme was used, and was not sufficient. Therefore, a Lewis acid catalyst that has an excellent selectivity and reactivity, and further capable of reacting under warm condition has been required.

Heretofore, a Lewis acid catalyst comprised of a compound shown by a general formula M[RfSO₂-N-SO₂Rf']ₙ or M[RfSO₂-N-SO₂Rf']ₙ·mH₂O (wherein Rf and Rf' represent a perfluoroalkyl group having 1 to 8 carbon atoms, M represents an element selected from alkal metal, alkaline earth metal, transition metal, rare earth, aluminum, gallium, iridium, thallium, silicon, germanium, tin, lead, arsenic, antimony, bismuth, selenium and tellurium, n represents an integer of the same number as the valence of the corresponding metal, and m represents a natural number from 0.5 to 20) (JP-A-7 246 338 (1995)), and a Lewis acid catalyst shown by the following formula, 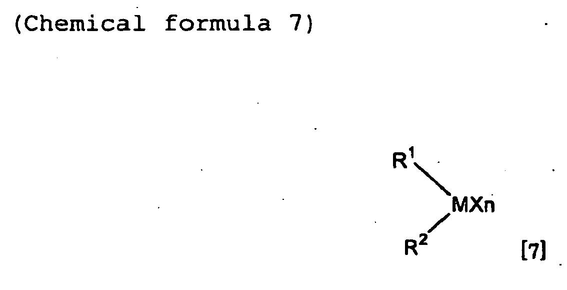 [wherein X represents -N(Tf¹)Tf² [wherein Tf¹ represents -SO₂Rf¹, Tf² represents -SO₂Rf² (wherein each of Rf¹ and Rf² independently represents a fluorine atom or a perfluoroalkyl group)], R¹ represents a substituted or unsubstituted cyclopentadienyl group, -OR³ or -N(Tf³)R⁴, R² represents a substituted or unsubstituted cyclopentadienyl group, -OR⁵ or -N(Tf⁴)R⁶ [wherein Tf³ represents -SO₂Rf³, Tf⁴ represents -SO₂Rf⁴ (wherein each of Rf³ and Rf⁴ independently represents a fluorine atom or a perfluoroalkyl group), each of R³, R⁴, R⁵ and R⁶ independently represents a lower alkyl group, or, R³ and R⁵, R³ and R⁶, R⁴ and R⁵ or R⁴ and R⁶ form together a bivalent group], M represents an element selected from alkaline earth metal, rare earth element, transition metal, boron, aluminum, gallium, indium, thallium, silicon, germanium, tin, lead, arsenic, antimony, bismuth, selenium or tellurium, n represents an integer of valence -2 of the corresponding M, and has at least one of -N(Tf¹)Tf², -N(Tf³)R⁴ or -N(Tf⁴)R⁶] (JP-A-9057110 (1997)), have been known as Lewis acid catalysts.

Aside from the examples mentioned above, there have been disclosures of highly active acid catalysts, including a highly active Lewis acid catalyst that can be used under the coexistence of water, comprising a metallic halide shown by a general formula M⁺(X₁⁻)_{q} (wherein M represents at least one metal selected from a group comprising elements from IIIA family to VB family of the periodic table, X₁ represents a halogen atom, and q represents an integer that is identical to the valence number of M) and a quaternary salt type anion exchange resin (JP-A-9262479 (1997)), and an acid catalyst comprising a metallic salt of tris(perfluoroalkylsulfonyl)methide shown by the following formula [(RfSO₂)₃C]ₙM₂ (however, Rf represents a perfluoroalkyl group having one or more carbon atoms, M₂ represents an element elected from an alkali metal, an alkaline earth metal, a transition metal including rare earth, zinc, cadmium, aluminum, gallium, indium, thallium, silicon, germanium, tin, lead, arsenic, antimony, bismuth, selenium or tellurium. n represents an integer of the same number as the valence of M₂) (JP-A-219692 (2000)).

Heretofore, TfOH, Tf₂NH, Tf₂CH₂ and Tf₃CH have already been known as an organic acid having a triflyl group. However, chemical modification to these molecules is not easy and therefore, it is difficult to make the molecules have a novel function. Due to this reason, they were not considered to be an appropriate material when producing pharmaceuticals, agricultural chemicals, asymmetric catalysts, various types of functional materials and the like. Moreover, recently, the development of a catalyst capable of easily conducting asymmetric synthesis, which is called chiral technology or chiral industry in the field of medicine and agricultural chemical, is expected for the development and application of pharmaceuticals, agricultural chemicals, various types of functional materials and the like.

The object of the present invention is to provide: a method wherein an arylbis(perfluoroalkylsulfonyl)methane such as the various types of arylbis(triflyl)methane and the like having a bulky aryl group and an electron-accepting aryl group in which its synthesis was conventionally considered to be difficult, is produced easily and highly efficiently; a novel arylbis(perfluoroalkylsulfonyl)methane such as pentafluorophenylbis(triflyl)methane, {4-(pentafluorophenyl)-2,3,5,6-tetrafluorophenyl}bis(triflyl)methane and the like, which can be widely applied to asymmetric catalyst, various types of functional materials and the like; a method for producing a metallic salt of arylbis(perfluoroalkylsulfonyl)methane that has excellent selectivity and reactivity, can further react under warm condition, and introduce various types of aryl groups, that is, metallic arylbis(perfluoroalkylsulfonyl)methide, wherein said arylbis(perfluoroalkylsulfonyl)methane is used,; a metallic arylbis(perfluoroalkylsulfonyl)methide obtained by said method; catalysts comprised of said compound and their use as Lewis acid catalysts; and a method for synthesizing organic compounds by using said catalysts.

### Disclosure of the Invention

The present inventors have found out that arylbis(trifluoromethylsulfonyl)methanes can be produced at a high yield by the following steps: an easily obtainable aryl halomethane was used as a starting material; sodium trifluoromethane sulfinate (TfNa) was used as an electrophilic reactant as a triflyl source; heating under reflux was conducted with the use of propionitrile as a solvent under the presence of 10 mol% tetrabutyl ammonium iodide catalyst to conduct nucleophilic substitution; arylmethyl trifluoromethylsulfone was produced at a high yield; and 2.2 equivalent weight of tert-butyl lithium (t-BuLi) and 1.1 equivalent weight of trifluoromethane sulfonic acid anhydride (Tf₂O) were sequentially added to the arylmethyl trifluoromethylsulfone produced. Thus, the present invention had been completed.

Further, the present inventors conducted a keen study to elucidate the object mentioned above. In the same manner as the method described above, TfNa and Tf₂O were used to synthesize pentafluorophenylbis(triflyl)methane; the pentafluorophenylbis(triflyl)methane obtainedwas subjected to heating under reflux with scandium oxide (Sc₂O₃) in water to produce scandium (III) pentafluorophenylbis(triflyl); said compound was used as a Lewis acid catalyst for benzoylation reaction with menthol and benzoic acid anhydride, and Diels-Alder reaction of methacrolein with cyclopentadiene; and it was confirmed that said compound has a better catalyst activity than the existing Lewis acids. Thus, the present invention has been completed.

The present invention relates to an arylbis(perfluoroalkylsulfonyl)methane represented by the following general formula [1] 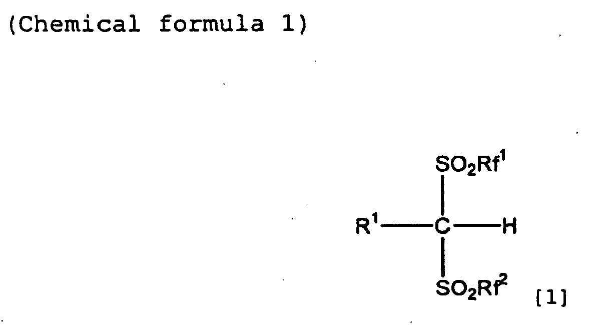 (wherein R¹ is a 2,4,6-trimethylphenyl group, a 4-(trifluoromethyl)phenyl group, a 3,5-bis(trifluoromethyl)phenyl group, a pentafluorophenyl group or a para substituted derivative thereof or a perfluorobiphenyl group or a 4' -substituted derivative thereof and Rf¹ and Rf² are independent to each other and represent a perfluoroalkyl group) (claim 1); the arylbis(perfluoroalkylsulfonyl)methane represented by the general formula [1] as defined above, wherein Rf¹ and Rf² are both a trifluoromethyl group (claim 2); a pentafluorophenylbis(trifluoromethylsulfonyl)methane represented by formula [2] 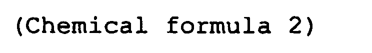 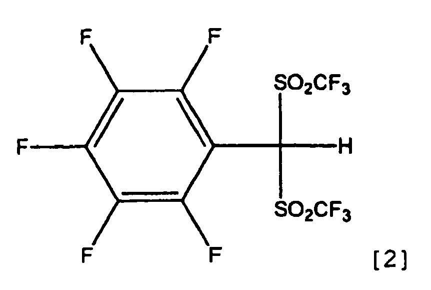 or a para substituted derivative thereof (claim 3); and a {4-(pentafluorophenyl)-2,3,5,6-tetrafluorophenyl}bis(trifly 1)methane represented by formula [3] 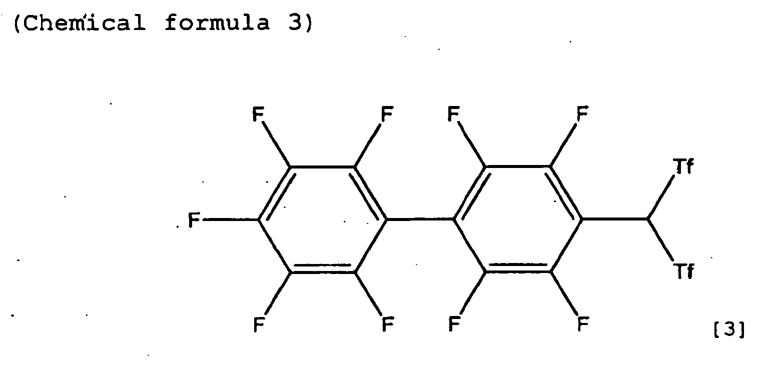 or a 4' substituted derivative thereof (claim 4).

Further, the present invention relates to a method for producing an arylbis(perfluoroalkylsulfonyl)methane represented by general formula [1] as defined above, wherein an aryl halomethane is reacted with a perfluoroalkyl sulfinate, the arylmethylperfluoroalkylsulfone produced is reacted with a deprotonation agent comprising an organic metal or a metallic salt, and the metallic salt of the arylmethylperfluoroalkylsulfone obtained is reacted with a perfluoroalkyl sulfonic acid anhydride (claim 5); the method described above, wherein the aryl halomethane is reacted with the perfluoroalkyl sulfinate by heating under reflux using a solvent with or without the presence of a catalyst (claim 6) ; the method described above, wherein tetrabutyl ammonium iodide is used as the catalyst (claim 7); the method described above wherein propionitrile is used as the solvent (claim 8); the method described above, wherein a deprotonation agent comprising 1.7 to 2.4 equivalent weight of an organic metal or a metallic salt is reacted with the arylmethylperfluoroalkylsulfone (claim 9); the method described above, wherein the aryl halomethane is 2,4,6-trimethylphenylmethyl chloride, 4-(trifluoromethyl)phenylmethyl bromide, 3,5-bis(trifluoxomethyl)phenylmethyl bromide, pentafluorophenylmethyl bromide or 4-(bromomethyl)perfluorobiphenyl (claim 10); the method described above, wherein the perfluoroalkyl sulfinate is an alkali metallic salt of trifluoromethane sulfinic acid (claim 11); and the method described above, wherein the metallic salt of arylmethylperfluoroalkylsulfone is a lithium salt or a magnesium salt of arylmethylperfluoroalkylsulfone (claim 12).

The present invention further relates to a metallic salt of arylbis(perfluoroalkylsulfonyl)methane represented by general formula [6] 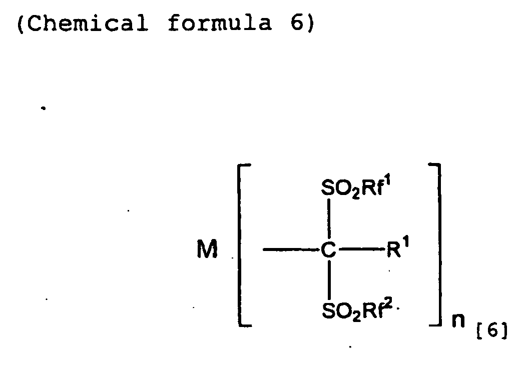 (wherein R¹, Rf¹ and Rf² are as defined above in relation to general formula [1], M represents any one of the elements selected from an alkali metallic element, an alkaline earth metallic element, a transition metallic element, boron, silicon, aluminum, tin, zinc or bismuth, and n shows a numeric value equal to the valence of M element) (claim 13); the metallic salt of arylbis(perfluoroalkylsulfonyl)methane as defined above, wherein the transition metallic element is any one of the metallic elements selected from scandium, yttrium, lanthanoid, copper, silver, titanium, zirconium or hafnium (claim 14); the metallic salt of arylbis(perfluoroalkylsulfonyl)methane as defined above,
wherein the metallic salt of arylbis(perfluoroalkylsulfonyl)methane is a metallic salt of 2,4,6-trimethylphenylbis(triflyl)methane, a metallic salt of 4-(trifluoromethyl)phenylbis(triflyl)methane, a metallic salt of 3,5-bis(trifluoromethyl)phenylbis(triflyl)methane, a metallic salt of pentafluorophenylbis(triflyl)methane or a metallic salt of {4-(pentafluorophenyl)-2,3,5,6-tetrafluorophenyl}bis(trifly 1)methane (claim 15); the above metallic salt of arylbis(perfluoroalkylsulfonyl)methane, wherein the metallic salt of pentafluorophenylbis(triflyl)methane is a scandium (III) pentafluorophenylbis(triflyl)methide or a lithium pentafluorophenylbis(triflyl)methide (claim 16); and the above metallic salt of arylbis(perfluoroalkylsulfonyl)methane, wherein the metallic salt of {4-(pentafluorophenyl)-2,3,5,6-tetrafluorophenyl}bis(trifly 1)methane is a scandium (III) {4-(pentafluorophenyl)-2,3,5,6-tetrafluorophenyl}bis(trifly 1)methide or a lithium {4-(pentafluorophenyl)-2,3,5,6-tetrafluorophenyl}bis(trifly 1)methide (claim 17).

Further, the present invention relates to a method for producing a metallic salt of arylbis(perfluoroalkylsulfonyl)methane represented by general formula [6], as defined above, wherein an arylbis(perfluoroalkylsulfonyl)methane represented by general formula [1], as defined above, is neutralized with a hydroxide of a metal (claim 18); the above method wherein the hydroxide of the metal is a hydroxide of a metal selected from an alkali metal or an alkaline earth metal (claim 19) ;
a method for producing a metallic salt of arylbis(perfluoroalkylsulfonyl)methane, represented by general formula [6'], as defined below, 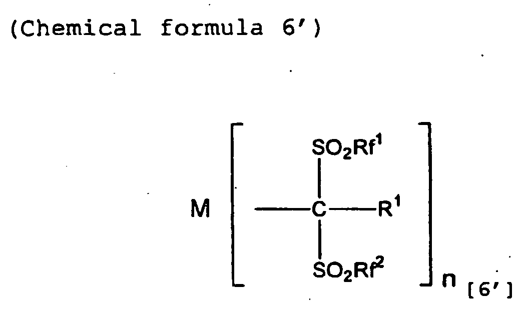 (wherein R¹, Rf¹ and Rf² are as defined above in relation to general formula [1], M is a transition metallic element, and n represents a numeric value equal to the valence of M element), wherein an arylbis(perfluoroalkylsulfonyl)methane represented by general formula [1], as defined above, is reacted with a salt or an oxide of transition metal by heating under reflux (claim 20); and the above-described method for producing the transition metallic salt of arylbis(perfluoroalkylsulfonyl)methane, wherein the salt or the oxide of transition metal is a lanthanoid metallic salt or a scandium oxide (claim 21).

The present invention also relates to a method for producing a metallic salt of arylbis (perfluoroalkylsulfonyl)methane represented by general formula [6], as defined above, wherein a metallic salt of arylbis (perfluoroalkylsulfonyl)methane represented by general formula [1], as defined above, is reacted with a halide of a metal having different metal species (claim 22); and the above-described method, wherein the metallic salt of arylbis (perfluoroalkylsulfonyl) methane represented by the general formula [6] is a silver salt of arylbis(perfluoroalkylsulfonyl)methane (claim 23).

Further, the present invention relates to a method for producing a silver salt of arylbis(perfluoroalkylsulfonyl)methane represented by general formula [6''] 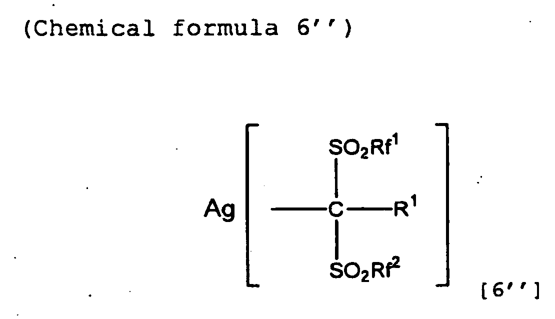 wherein an arylbis(perfluoroalkylsulfonyl)methane represented by general formula [1], either in its broadest aspect or a preferred aspect, is reacted with silver carbonate under shade (claim 24); the method for producing the metallic salt of arylbis(perfluoroalkylsulfonyl)methane represented by the general formula [6], [6'] or [6''] by reacting an aryl halomethane with a perfluoroalkyl sulfinate, followed by reacting the arylmethylperfluoroalkylsulfone produced with a deprotonation agent comprising an organic metal or a metallic salt, and the metallic salt of the arylmethylperfluoroalkylsulfone obtained is reacted with a perfluoroalkyl sulfonic acid anhydride (claim 25); the above-described method, wherein the aryl halomethane is reacted with the perfluoroalkyl sulfinate by heating under reflux using a solvent with or without the presence of a catalyst (claim 26); the above-described method, wherein tetrabutyl ammonium iodide is used as the catalyst (claim 27); the above-described method for producing the metallic salt of arylbis(perfluoroalkylsulfonyl)methane wherein propionitrile is used as the solvent (claim 28); the above-described method wherein the perfluoroalkyl sulfinate is an alkali metallic salt of trifluoromethane sulfinic acid (claim 29); and the above-described method, wherein the metallic salt of arylmethylperfluoroalkylsulfone is a lithium salt or a magnesium salt of arylmethylperfluoroalkylsulfone (claim 30).

Moreover, the present invention relates to a catalyst having a metallic salt of arylbis(perfluoroalkylsulfonyl)methane represented by general formula [6], as defined above, as an active ingredient (claim 31) ; and use of the above catalyst as a Lewis acid catalyst (claim 32).

Still further, the present invention relates to a method for synthesizing an organic compound using a catalyst having a metallic salt of arylbis(perfluoroalkylsulfonyl)methane represented by general formula [6], as defined above, as an active ingredient, wherein the catalytic reaction is conducted under the presence of said catalyst in a solvent, wherein the catalytic reaction is a benzoylation reaction or a Diels-Alder reaction (claim 33).

### Best Mode of Carrying Out the Invention

In the arylbis(perfluoroalkylsulfonyl)methane represented by the general formula [1], R¹ is a 2,4,6-trimethylphenyl group, a 4-(trifluoromethyl)phenyl group, a 3,5-bis(trifluoromethyl)phenyl group, a pentafluorophenyl group or a para-substituted derivative thereof, or a perfluorobiphenyl group or a 4'-substituted derivative thereof, and Rf¹ and Rf² are independent to each other and represent a perfluoroalkyl group. Examples of the substituent for this case include a C1-C4 alkyl group such as methyl group and the like, a halogenated C1-C4 alkyl group such as trifluoromethyl group and the like, a halogen atom such as fluorine and the like, an alkoxy group, a sulfonyl group, an amino group and the like.

There is no particular limitation to the method for producing arylbis(perfluoroalkylsulfonyl) methane represented by the general formula [1] (as defined above) of the present invention, as long as it is a method wherein an aryl halomethane is reacted with a perfluoroalkyl sulfinate, the arylmethylperfluoroalkylsulfone produced is reacted with a deprotonation agent comprising an organic metal or a metallic salt, and the metallic salt of the arylmethylperfluoroalkylsulfone obtained is reacted with an anhydrous perfluoroalkyl sulfonic acid. Moreover, the method for producing an arylbis(perfluoroalkylsulfonyl)methane obtained by further reacting the arylbis(perfluoroalkylsulfonyl)methane obtained by the method mentioned above with alkyl anion such as alkyl lithium, alkoxy anion such as alkoxy lithium and the like, is also included in the method of the present invention.

The Rf¹ and Rf² in the general formula [1] mentioned above represent a perfluoroalkyl group that may be the same or different from each other, preferably a C1-C8 perfluoroalkyl group. Specific examples of -SO₂Rf¹ and -SO₂Rf² containing these Rf¹ and Rf² include a trifluoromethylsulfonyl group, a perfluoroethylsulfonyl group, a perfluoropropylsulfonyl group, a perfluoroisopropylsulfonyl group, a perfluorobutylsulfonyl group, a perfluoroisobutylsulfonyl group, a perfluoropentylsulfonyl group, a perfluoroisopentylsulfonyl group, a perfluoroneopentylsulfonyl group and the like.

Specific examples of the arylbis(perfluoroalkylsulfonyl)methane represented by the general formula [1] of the present invention are, for example, 2,4,6-trimethylphenylbis(triflyl)methane, 4-(trifluoromethyl)phenylbis(triflyl)methane, 3,5-bis(trifluoromethyl)phenylbis(triflyl)methane, the pentafluorophenylbis(triflyl)methane represented by the formula [2], the {4-(pentafluorophenyl)-2,3,5,6-tetrafluorophenyl}bis(trifly 1) methane represented by the formula [3] and the like. However, the examples are not limited to these. Further, examples of the compound of the present invention include: a para substituted derivative of the pentafluorophenylbis(triflyl)methane represented by the formula [2] mentioned above, for example, a para position alkyl substituted derivative such as a p-phenyl-2,3,5,6-tetrafluorophenyl-bis(triflyl)methane and the like, a para position alkoxy substituted derivative such as p-hexyloxy-2,3,5,6-tetrafluorophenyl-bis(triflyl)methane and the like; a 4'-position substituted derivative of the {4-(pentafluorophenyl)-2,3,5,6-tetrafluorophenyl}bis(trifly 1)methane represented by the formula [3], for example, a 4'-position alkyl substituted derivative such as a [4-(4-phenyl-2,3,5,6-tetrafluorophenyl)-2,3,5,6-tetrafluoro phenyl]bis(triflyl)methane and the like, a 4'-position alkoxy substituted derivative such as a [4-(4-hexyloxy-2,3,5,6-tetrafluorophenyl)-2,3,5,6-tetrafluo rophenyl]bis(triflyl)methane and the like, etc.

There is no particular limitation to the aryl halomethane used in the method for producing the arylbis(perfluoroalkylsulfonyl)methane represented by the general formula [1] of the present invention, as long as it is a methane substituted by a substituted or unsubstituted aryl group (as defined above for R¹) and a halogen atom. Specific examples include 2,4,6-trimethylphenylmethyl chloride, 4-(trifluoromethyl)phenylmethyl bromide, 3,5-bis(trifluoromethyl)phenylmethyl bromide, pentafluorophenylmethyl bromide, 4-(bromomethyl)perfluorobiphenyl (perfluorobiphenylmethyl bromide) and the like.

A preferred example of the perfluoroalkyl sulfinate used in the method for producing the arylbis(perfluoroalkylsulfonyl)methane represented by the general formula [1] of the present invention is a metallic salt of a C1-C8 perfluoroalkyl sulfinic acid including, for example, a trifluoromethyl sulfinic acid, a perfluoroethyl sulfinic acid, a perfluoropropyl sulfinic acid, a perfluoroisopropyl sulfinic acid, a perfluorobutyl sulfinic acid, a perfluoroisobutyl sulfinic acid, a perfluoropentyl sulfinic acid, a perfluoroisopentyl sulfinic acid, a perfluoroneopentyl sulfinic acid and the like. An alkali metallic salt and an alkaline earth metallic salt can be exemplified as the metallic salt, however, an alkali metallic salt such as sodium and the like is preferable.

It is preferable for the nucleophilic substitution reactionof the aryl halomethane and the perfluoroalkyl sulfinate in the method for producing the arylbis(perfluoroalkylsulfonyl)methane represented by the general formula [1] of the present invention to be conducted in a condition wherein an arylmethylperfluoroalkylsulfone can be synthesized at a high efficiency, for example, by heating under reflux using a solvent with or without the presence of a catalyst. It is preferable for the polarity of the aryl halomethane in the reaction system mentioned above to be 0.2 to 0.4 M, and for the perfluoroalkyl sulfinate such as sodium trifluoromethane sulfinate to be used in an amount of 1.0 to 1.5 equivalent weight, especially about 1.3 equivalent weight, to the aryl halomethane. In addition, when a catalyst is used, the use of a catalyst comprising an iodide such as tetrabutyl ammonium iodide, potassium iodide and the like is preferable. The amount of these catalysts to be used is, for example, 2 to 20 mol%, preferably 5 to 10 mol% to the aryl halomethane. Further, acetonitrile, propionitrile, nitromethane, nitropropane and the like can be given as examples of the solvent, however, it is preferable to use propionitrile in view of the applicabiliity of the polarity and boiling point.

The synthesis reaction mentioned above is preferable to be conducted by heating under reflux in a dry inert gas atmosphere, such as in an argon or nitrogen atmosphere. It is preferable for the reaction to be conducted by heating under reflux at 80 to 150°C, preferably 100°C to 120°C for 12 to 48 hours. Examples of the methods for purifying the arylmethyl triflone obtained by these synthesis reactions are, for example, a method wherein the reactant solution obtained by reacting under the condition mentioned above is filtrated to remove salt, followed by a silica gel column chromatography using hexane and ethyl acetate (EtOAc) as a developing solvent, and a recrystallization operation using hexane and toluene, or the like.

Next, the arylbis(perfluoroalkylsulfonyl)methane represented by the general formula [1] can be produced by the reaction of an arylmethylperfluoroalkylsulfone produced by the nucleophilic substitution reaction of aryl halomethane and perfluoroalkyl sulfinate with a deprotonation agent comprising an organic metal or a metallic salt, followed by the reaction of the metallic salt of arylmethylperfluoroalkylsulfone obtained with a perfluoroalkyl sulfonic acid anhydride. However, there is no particular limitation to the deprotonation agent mentioned above, as long as it is an organic metal or a metallic salt having a deprotonating action. An alkali metallic salt and alkaline earth metallic salt of lower alkyl, more specifically, t-BuLi and t-BuMgCl can preferably be exemplified. Further, a preferable example of the perfluoroalkyl sulfonic acid anhydride mentioned above is a C1-C8 perfluoroalkyl sulfonic acid anhydride including trifluoromethane sulfonic acid anhydride (Tf₂O), perfluoroethane sulfonic acid anhydride, perfluoropropane sulfonic acid anhydride, perfluoroisopropane sulfonic acid anhydride, perfluorobutane sulfonic acid anhydride, perfluoroisobutane sulfonic acid anhydride, perfluoropentane sulfonic acid anhydride, perfluoroisopentane sulfonic acid anhydride, perfluoroneopentane sulfonic acid anhydride and the like. Among these, Tf₂O is especially preferable.

There is no particular limitation to the method wherein the arylmethylperfluoroalkylsulfone mentioned above is reacted with a deprotonation agent such as alkyl lithium, alkyl magnesium chloride and the like and a perfluoroalkyl sulfonic acid anhydride such as Tf₂O and the like, as long as it is a method which can produce the arylbis(perfluoroalkylsulfonyl)methane, such as arylbis (trifluoromethylsulfonyl) methane and the like, at a high yield. Specific examples include, for example: a method wherein an arylmethylperfluoroalkylsulfone such as arylmethyl triflone and the like is dissolved in a solvent such as diethyl ether and the like, alkyl lithium is added at -78°C, the solvent is reacted for 5 to 10 minutes, then Tf₂O is added after the reaction to react for 1 to 2 hours at room temperature; and a method wherein alkyl magnesium chloride is added at-78°C to react for 30 minutes, then reacted at 0°C for 30 minutes, and Tf₂O is added at -78°C after the reaction to react for 1 to 2 hours at room temperature, and the like. However, it is preferable to repeat said operation multiple times, in view of the increase in yield.

Further, in order to obtain an arylbis(perfluoroalkylsulfonyl)methane such as arylbis(trifluoromethylsulfonyl)methane at a high yield, it is preferable to react 1.7 to 2.4 equivalent weight of an organic metal such as alkyl lithium and the like or 1.0 to 1.2 equivalent weight of a perfluoroalkyl sulfonic acid anhydride such as Tf₂O and the like with the arylmethylperfluoroalkylsulfone such as arylmethyl triflone and the like.

However, in the case where a pentafluorophenylbis(triflyl)methane is produced by using a pentafluorophenylmethyl bromide, a pentafluorophenylbis(triflyl)methane and a 4-tert-butyl-2,3,5,6-tetrafluorophenylbis(triflyl)methane are both obtained at a ratio of 1:1 (45% yield, respectively). Therefore, in this case, using 1.0 equivalent weight of t-BuLi and 0.5 equivalent weight of Tf₂O completely suppresses the production of 4-tert-butyl-2,3,5,6-tetrafluorophenylbis(triflyl)methane, and a pentafluorophenylbis (triflyl)methane with Tf₂O as a base can be obtained at a high yield.

In the metallic salt of the arylbis(perfluoroalkylsulfonyl)methane represented by the general formula [6] of the present invention [sometimes referred to as metallic arylbis(trifluoromethylsulfonyl)methide], R¹, Rf¹ and Rf² are as defined above for formula [1], and M represents any one of the elements selected from an alkali metallic element, an alkaline earth metallic element, a transition metallic element, silicon, germanium, tin, lead, arsenic, antimony, bismuth or tellurium, and n shows a numeric value equal to the valence of M element. Here, examples of the metal species of metallic arylbis(trifluoromethylsulfonyl)methide include, for example: alkali metallic elements such as lithium, sodium, potassium, rubidium, cesium and francium; alkaline earth metallic elements such as beryllium, magnesium, calcium, strontium, barium and radium; transition metallic elements such as scandium, yttrium, lanthanum, cerium, praseodymium, neodymium, promethium, samarium, europium, gadolinium, terbium, dysprosium, holmium, erbium, thulium, ytterbium, lutetium, titanium, zirconium, hafnium, vanadium, niobium, tantalum, chromium, molybdenum, tungsten, manganese, technetium, rhenium, iron, ruthenium, osmium, cobalt, rhodium, iridium, nickel, palladium, boron, aluminum, platinum, copper, silver, gold, zinc, cadmium and mercury; silicon; germanium; tin; lead; arsenic; antimony; bismuth or tellurium. Among these, lithium, scandium, silver, silicon and the like are especially preferable.

The R¹ in the formula [6] mentioned above is the same as the R¹ represented by the general formula [1] in the arylbis(perfluoroalkylsulfonyl)methane of the present invention. The Rf¹ and Rf² in the general formula [6] mentioned above is the same as Rf¹ and Rf² in the arylbis(perfluoroalkylsulfonyl)methane represented by the general formula [1] of the present invention.

Specific examples of the metallic arylbis(perfluoroalkylsulfonyl)methide of the present invention include, for example, a metallic salt of 2,4,6-trimethylphenylbis(triflyl)methane, a metallic salt of 4- (trifluoromethyl) phenylbis (triflyl)methane, a metallic salt of 3,5-bis(trifluoromethyl)phenylbis(triflyl)methane, a metallic salt of pentafluorophenylbis(triflyl)methane, a metallic salt of {4-(pentafluorophenyl)-2,3,5,6-tetrafluorophenyl}bis(trifly 1)methane, and the like. Among these, the metallic salts such as lithium salt, scandium salt and the like are preferable, and the scandium salt and lithium salt of pentafluorophenylbis(triflyl)methane and {4-(pentafluorophenyl)-2,3,5,6-tetrafluorophenyl}bis(trifly 1)methane are especially preferable in view of the catalytic activity and the like.

As to the method for producing the metallic arylbis(trifluoromethylsulfonyl)methide of the present invention, for example, the reactions of the arylbis(trifluoromethylsulfonyl)methane of the present invention mentioned above can be given as follows: (1) neutralization with a hydroxide of a metal, (2) reaction by heating under reflux with a salt or an oxide of a transition metal, and (3) reaction with a silver carbonate under shade. Further, another method which is the exchange reaction of metal species, can be exemplified, wherein a metallic salt such as a silver salt of the arylbis(perfluoroalkylsulfonyl)methane represented by the general formula [1], and a halide of a metal of different metal species are reacted. Specific examples of the hydroxide of the metal in the neutralization in (1) mentioned above include hydroxides of alkali metals such as lithium hydroxide, sodium hydroxide and potassium hydroxide, and hydroxides of alkaline earth metal such as calcium hydroxide. A method wherein said arylbis (trifluoromethylsulfonyl) methane of the present invention is reacted for 10 minutes to over 10 hours by using a solution wherein the hydroxides of these metals are dissolved in a solvent such as diethyl ether and the like, can be exemplified. Specific examples of the salt or the oxide of the transition metal in the reaction by heating under reflux in (2) mentioned above include the lanthanoid metallic salt such as the chloride and the like of lanthanum and cerium, and scandium oxide such as Sc₂O₃ and the like. A method wherein heating under reflux in the aqueous solution is conducted for 10 minutes to over 10 hours can be exemplified.

The R¹ and Rf¹ and Rf² in the arylbis(perfluoroalkylsulfonyl)methane represented by the general forumula [1] in the method for producing the metallic arylbis(trifluoromethylsulfonyl)methide of the present invention represents the same groups as that of R¹ and Rf¹ and Rf² in the general formula [6] mentioned above. As a method for producing an arylbis(perfluoroalkylsulfonyl)methane, the method for producing the arylbis(trifluoromethylsulfonyl)methane of the present invention can be applied.

There is no limitation to the catalyst (or its use as a Lewis acid catalyst) and the like comprising the metallic salt of arylbis(perfluoroalkylsulfonyl)methane of the present invention, as long as it is a catalyst that at least comprises a metallic salt of arylbis(perfluoroalkylsulfonyl)methane as defined in general formula [6] above as an active ingredient. For example, a catalyst that is supported on a carrier, a catalyst that is fixed on a high polymer compound, and a catalyst having a surface active-ability by making a hydrophobic atomic group and a hydrophilic atomic group exist in the molecules, can be given as specific examples. These catalysts (preferably used as Lewis acid catalyst) and the like comprising the metallic salt of arylbis(perfluoroalkylsulfonyl)methane have a catalytic activity that exceeds that of the existing Lewis acids, and therefore, they can be used for synthesis reaction of organic compounds in a high yield and with excellent selectivity.

The use of the catalyst such as Lewis acid catalyst and the like comprising the metallic salt of the arylbis(perfluoroalkylsulfonyl)methane mentioned above makes it possible to synthesize organic compounds such as pharmaceuticals, agricultural chemical, asymmetric catalyst, various types of functional materials, and the like. A specific example of said method for synthesizing is a method wherein a catalytic reaction is conducted under the presence of a catalyst such as Lewis acid catalyst or the like comprising the metallic salt of arylbis(perfluoroalkylsulfonyl)methane mentioned above in an aqueous solution, in an organic solvent or in a mixed solvent of water and organic solvent. Specific examples of the catalytic reaction mentioned above include a benzoylation reaction, a Diels-Alder reaction, an aldol-type reaction, a Friedel-Crafts reaction, a Mannich reaction, a glycosilation reaction, an esterification reaction, an ene reaction, a cationic polymerization reaction, an allylation reaction, an interesterification reaction, a Mannich-type reaction, a Michael addition reaction, an acylation reaction, a conjugate addition reaction, a dehydration reaction, a dehydration/condensation reaction, a polymerization reaction and the like.

The present invention will now be explained further in more details with the examples below, however, the scope of the invention is not limited to the exemplifications.

### Example 1 [Analysis and Material]

The infrared radiation spectrum was determined by using a Shimadzu FTIR-9100. The ¹H NMR spectrum was determined by using a Varian Gemini-300 (300 MHz) nuclear magnetic resonance apparatus. The chemical shift of ¹H NMR was indicated by ppm wherein a solvent as an internal standard (tetramethylsilane at 0 ppm) was used. The division pattern was shown as singlet: s, doublet: d, triplet: t, quartet: q, multiplet: m and broad peak: br. The ¹³C NMR spectrum was determined by using a Varian Gemini-300 (125 MHz) nuclear magnetic resonance apparatus, and was indicated by ppm wherein a solvent as an internal standard (CDCl₃ at 77.0 ppm) was used. The ¹⁹F NMR spectrum was determine by using a Varian Gemini-300 (282 MHz) nuclear magnetic resonance apparatus, and was indicated by ppm wherein a solvent as an internal standard (CF₃C₆H₅ at -64.0 ppm) was used. High-performance liquid chromatography (HPLC) analysis was conducted with a Shimadzu LC-10AD instrument and an SPD-M10A UV detector by using a chiral column (Daicel, AS or OD-H). All of the following examples were conducted in a glass instrument dried in an oven, by using a magnetic stirrer. The reaction product was purified on a Silica Gel E. Merck 9385 or a Silica Gel 60 Extra Pure by flash chromatography. High resolution mass spectrometry (HRMS) analysis was performed with the use of an instrument of Daikin Industries, Ltd.

### Example 2 [Synthesis of Arylmethyl Triflone]

Each of the mixed solutions of aryl halomethyl (10 mmol), sodium trifluoromethane sulfinate (2.0 g: 13 mmol), propionitrile (30 mL) and tetrabutyl ammonium iodide (0.37 g: 1 mmol), shown in Table 1, were subjected to heating under reflux in an argon atmosphere for approximately 1 day. After the heating under reflux, the reaction solutions were cooled to room temperature, and were concentrated after removing the salt by filtration. The crude products obtained were purified by silica gel column chromatography (developing solvent: hexane-EtOAc) or recrystallization operation (hexane-toluene) to obtain arylmethyl triflone. The yield of each of the arylmethyl triflone is indicated in Table 1, and the physical property of each of the arylmethyl triflone is shown below. Table 1 showed that when sodium trifluoromethane sulfinate (TfNa) used as an electrophilic reactant as a triflyl source, and heating under reflux with aryl halomethane was conducted by using propionitrile as a solvent under the presence of a tetrabutyl ammonium iodide catalyst, arylmethyl triflone can be obtained at a high yield than the method of Hendrickson et al. (Synthesis, 691, 1997).

**Table 1**

| aryl halomethane | arylmethyl triflone | [yield (%)] |
|---|---|---|
| 2,4,6-Me₃C₆H₂CH₂Cl | 2,4,6-Me₃C₆H₂CH₂Tf | 90 |
| 4-CF₃C₆H₄CH₂Br | 4-CF₃C₈H₄CH₂Tf | >99 |
| 3,5-(CF₃)₂C₆H₃CH₂Br | 3,5-(CF₃)₂C₆H₄CH₂Tf | 76 |
| C₆F₅CH₂Br | C₆F₅CH₂Tf | 89 |

2,4,6-trimethylphenylmethyl triflone (2,4,6-Trimethylphenylmethyl triflone): IR (KBr) 1358, 1206, 1117, 864, 619, 550, 500, 469 cm⁻¹ ; ¹H NMR (CDCl₃, 300 MHz) δ 2.29 (s, 3H), 2.43 (s, 6H) 4.62 (s, 2H), 6.96 (s, 2H) ; ¹³C NMR (CDCl₃, 125 MHz) δ 20. 3, 21.0 (2C) , 49. 8, 117.0, 120.0 (q, J_{CF} = 326 Hz, 1C, CF₃), 129.9 (2C), 139.7 (2C), 139.8; ¹⁹F NMR (CDCl₃, 282 MHz) δ -79.7 (s, 3F, CF₃). Anal. Calcd. for C₁₁H₁₃O₂F₃S: C, 49.62; H, 4.92; F, 21.40; S, 12.04. Found C, 49.58; H, 4.53; F, 21.35; S, 12.06.

4-(trifluoromethyl)phenylmethyl triflone (4-(Trifluoromethyl)phenylmethyl Triflone; Synthesis, 691, 1997): IR (KBr) 1356, 1341, 1227, 1210, 1144, 1121, 855, 658, 513 cm⁻¹; ¹H NMR (CDCl₃, 300 MHz) δ 4.53 (s, 2H), 7.58 (d, J = 8.0 Hz, 2H), 7.72 (d, J = 8.0 Hz, 2H); ¹⁹F NMR (CDCl₃, 282 MHz) 6 -77.5 (s, 3F, CF₃), -64.3 (s, 3F, CF₃).

3,5-bis(trifluoromethyl)phenylmethyl triflone (3,5-Bis(trifluoromethyl)phenylmethyl Triflone): IR (KBr) 1376, 1362, 1277, 1175, 1117, 918, 910, 669 cm⁻¹; ¹H NMR (CDCl₃, 300 MHz) δ 4.60 (s, 2H), 7.91 (s, 2H), 8.01 (s, 1H); ¹³C NMR (CDCl₃, 125 MHz) δ 55.0, 119.6 (q, J_{CF} = 326 Hz, 1C, CF₃), 122.6 (q, J_{CF} = 272 Hz, 2C, 2CF₃), 124.2 (septet, J_{CF} = 4 Hz, 1C), 126.1, 131.3 (2C), 132.9 (q, J_{CF} = 34 Hz, 2C); ¹⁹F NMR (CDCl₃, 282 MHz) δ-77.4 (s, 3F, CF₃), -64.3 (s, 6F, 2CF₃). Anal. Calcd. for C₁₀H₃O₂F₉S: C, 33.53; H, 0.84; F, 47.74; S, 8.95. Found C, 33.48; H, 0.91; F, 47.87; S, 8.89.

Pentafluorophenylmethyl triflone (Pentafluorophenylmethyl Triflone) : IR (kBr) 1509, 1374, 1210, 1121, 995 cm⁻¹; ¹H NMR (CDCl₃, 300MHz) δ 4.64; ¹³C NMR (CDCl₃, 125 MHz) δ 44.3, 100.0 (dt, J_{CF} = 4, 17 Hz, 1C, ipso-C), 119.5 (q, J_{CF} = 326 Hz, 1C, CF₃), 137.9 (d, J_{CF} = 251 Hz, 2C, 2m-C), 142.8 (d, J_{CF} = 258 Hz, 1C, p-C), 145.9 (d, J_{CF} = 252 Hz, 2C, 2o-C); ¹⁹F NMR (CDCl₃, 282 MHz) δ-160.0 (d, J = 15.2 Hz, 2F, 2m-F), 149.0 (s, 1F, p-F), 139.4 (d, J = 15.2 Hz, 2F, 2o-F), -78.3 (s, 3F, CF₃). Anal. Calcd. for C₈H₂O₂F₆S: C, 30.59; H, 0.64; F, 48.38; S, 10.21. Found C, 30.49; H, 0.73; F, 48.37; S, 10.18. Example 3 (Reference) [Examinationof the Method for Synthesizing Arylbis(triflyl)methane]

The benzyl triflone obtained from Example 2 (0.5 mmol) was dissolved in diethylether (3 mL), this solution was cooled to -78°C, then added with 2.2 equivalent weight (1.1 mmol) of t-BuLi (0.34 mL, 1.6 M pentane solution), and was stirred for 0.5 hour. Next, after Tf₂O (46 µL, 0.55 mmol) was added, the temperature of the reaction solution was raised to room temperature, and the solution was further stirred for 1 hour. Subsequently, water was added to stop the reaction, the solution was neutralized, and then washed with hexane. These aqueous phases were acidified with 4 M of hydrochloride, and were twice extracted with diethylether. The organic phase was dried, filtrated and concentrated with magnesium sulfate to obtain phenylbis(triflyl)methane [PhCHTf₂] as a solid (79% yield). However, only a small amount of phenyltris(triflyl)methane [PhCTf₃] was produced. Meanwhile, the same reaction as mentioned above, except for the use of 1.1 equivalent weight of t-BuLi instead of 2.2 equivalent weight of t-BuLi, was conducted, and the yield of PhCHTf2 was 6% and that of PhCTf₃ was 46%.

### Example 4 [Synthesis of Arylbis(triflyl)methane]

Each of the arylmethyl triflones (0.5 mmol) obtained from Example 2 were dissolved in diethylether (3 mL), and their solutions were prepared, respectively. These solutions were cooled to -78°C, then added with 1.1 equivalent weight (0.55 mmol) oft-BuLi (0.34mL, 1. 6 M pentane solution) , and were stirred for 10 minutes. Next, Tf₂O (46 µL, 0.275 mmol) was added, the temperature of the reaction solution was raised to room temperature, and the solution was further stirred for 1 hour. After cooling the solution again to -78°C, 1.1 equivalent weight (0.55 mmol) of t-BuLi (0.34 mL, 1. 6 M pentane solution) was added, and the solution was stirred for 10 minutes. Subsequently, Tf₂O (46 µL, 0.275 mmol) was added, the temperature of the reaction solution was raised to room temperature, and the solution was further stirred for 1 hour. Then, water was added to stop the reaction, the solution was neutralized, and then washed witch hexane. These aqueous phases were acidified with 4 M of hydrochloride, and were twice extracted with diethylether. The organic phase was dried, filtrated and concentrated with magnesium sulfate to obtain arylbis (triflyl)methane as a solid. No further purification was needed. The yield of each of the arylmethyl triflone is indicated in Table 2, and the physical property of each of the arylmethyl triflone is shown below.

**Table 2**

| arylmethyl triflone | arylbis(triflyl)methane | [yield (%)] |
|---|---|---|
| 2,4,6-Me₃C₆H₂CH₂Tf | 2,4,6-Me₃C₆H₂CHTf₂ | 89 |
| 4-CF₃C₆H₄CH₂Tf | 4-CF₃C₆H₄CHTf₂ | 87 |
| 3,5-(CF₃)₂C₆H₃CH₂Tf | 3,5-(CF₃)₂C₆H₃CHTf₂ | 75 |
| C₆F₅CH₂Tf | C₆F₅CHTf₂ | 45 |

2,4,6-trimethylphenylbis(triflyl)methane (2,4,6-Trimethylphenylbis(triflyl)methane): IR (KBr) 1397, 1383, 1217, 1119, 1107, 642, 590 cm⁻¹; ¹H NMR (CDCl₃, 300 MHz) δ 2.33 (s, 3H), 2.35 (s, 3H), 2.61 (s, 3H), 6.48 (s, 1H), 7.00 (s, 1H) , 7.08 (2, 1H) ; ¹³C NMR (CDCl₃, 75 MHz) δ 20.2, 21.1, 22. 2, 77.7, 115.9, 119.4 (q, J_{CF} = 328 Hz, 2C, 2CF₃), 130.4, 132.2, 140.0, 142.2, 142.6; ¹⁹F NMR (CDCl₃, 282 MHz) δ-76.3 (s, 6F, 2CF₃) ; HRMS (EI) calcd for C₁₂H₁₂O₄F₆S₂ [M]⁺ 398.0081, found 398.0089.

4-(trifluoromethyl)phenylbis(triflyl)methane (4-(Trifluoromethyl)phenylbis(triflyl)methane) : IR (KBr) 1393, 1383, 1327, 1231, 1171, 1136, 1111, 860, 671, 610 cm⁻¹; ¹H NMR (CDCl₃, 300 MHz) δ 5.98 (s, 1H), 7.84 (s, 4H) ; ¹³C NMR (CDCl₃, 125 MHz) δ 80.4, 120.0 (q, J_{CF} = 329 Hz, 2C, 2CF₃), 123.8 (q, J_{CF} = 271 Hz, 1C, CF₃), 124.2, 127.6 (q, J = 4 Hz, 2C) , 133.0 (2C), 135.6 (q, J_{CF} = 33 Hz, 1C); ¹⁹F NMR (CDCl₃, 282 MHz) δ -73.5 (s, 6F, 2CF₃), -64.7 (s, 3F, CF₃) ; HRMS (EI) calcd. for C₁₀H₅O₄F₉S₂ [M]⁺ 423.9486, found 423.9471.

3,5-bis(trifluoromethyl)phenylbis(triflyl)methane (3,5-Bis(trifluoromethyl)phenylbis(triflyl)methane): IR (KBr) 1395, 1374, 1285, 1223, 1194, 1179, 1144, 1105, 936, 909, 629, 519 cm⁻¹; ¹H NMR (CDCl₃, 300 MHz) δ 6.05 (s, 1H), 8.13 (s, 2H), 8.18 (s, 1H); ¹³C NMR (CDCl₃, 125 MHz) δ78.9, 119.2 (q, J_{CF} = 329 Hz, 2C, 2CF₃), 122.2 (q, J_{CF} = 272 Hz, 2C, 2CF₃), 122.9, 126.7 (septet, J_{CF} = 4 Hz), 131.6 (s, 2C), 133.8 (q, J = 35 Hz, 2C); ¹⁹F NMR (CDCl₃, 282 MHz) δ-73.2 (s, 6F, 2CF₃), -64.3 (s, 6F, 2CF₃); HRMS (EI) calcd for C₁₁H₄O₄F₁₂S₂ [M]⁺ 472.9375, found 472.9372.

Pentafluorophenylbis(triflyl) methane (Pentafluorophenylbis(triflyl)methane) : Mp. 86°C to 87°C; IR (KBr) 1522, 1501, 1347, 1321, 1198, 1127, 1024, 988, 613 cm⁻¹; ¹H NMR (CDCl₃, 300 MHz) δ 6.21 (brs, 1H) ; ¹³C NMR (CDCl₃, 125 MHz) δ 70.4, 98.0 (s, 1C, ipso-C), 119.2 (q, J_{CF} = 330 Hz, 2C, 2CF₃) 137.8 (d, J_{CF} = 258 Hz, 1C, m-C), 138.6 (d, J_{CF} = 257 Hz, 1C, m-C), 144.7 (d, J_{CF} = 264 Hz, 1C, p-C), 145.4 (d, J_{CF} = 262 Hz, 1C o-C), 147.2 (d, J_{CF} = 262 Hz, 1C, o-C) ; ¹³C NMR (CD₃OD (δ49.0), 125 MHz) δ56.2, 109.1 (dt, J = 6, 19 Hz, 1C, ipso-C), 122.4 (q, J_{CF} = 324 Hz, 2C, 2CF₃), 138.5 (d, J_{CF} = 250 Hz, 2C, 2m-C), 143.0 (d, J_{CF} = 251 Hz, 1C, p-C), 150.0 (d, J_{CF} = 245 Hz, 1C, o-C), ¹⁹F NMR (CDCl₃, 282 MHz) δ-157.9 (dt, J = 6.2, 21.5 Hz, 1F, m-F), -156.8 (dt, J = 6.2, 21.5 Hz, 1F, m-F), -142.6 (tt, J = 5.9, 21.5 Hz, 1F, p-F), -140.3 (br, 1F, o-F), -127.7 (ddd, J = 5.9, 15.2, 21.5 Hz, 1F, o-F), -75.2 (s, 6F, 2CF₃); HRMS (EI) calcd. for C₉HO₄F₁₁S₂ [M]⁺ 445.9141, found 445.9137.

### Example 5 [Nucleophilic Substitution Specific to the Para Position of Pentafluorophenylbis(triflyl)methane]

As it is described in Table 2 that the yield of pentafluorophenylbis(triflyl)methane is 45%, it was revealed that when pentafluorophenylbis(triflyl)methane is produced using a pentafluoromethylbromide, both of pentafluorophenylbis(triflyl)methane and 4-tert-butyl-2,3,5,6-tetrafluorophenylbis(triflyl)methane can be obtained at a ratio of 1:1 (45% yield, respectively). However, it was found out that when 1.0 equivalent weight of t-BuLi and 0.5 equivalent weight of Tf₂O are used, the production of 4-tert-butyl-2,3,5,6-tetrafluorophenylbis(triflyl)methane is completely suppressed, and pentafluorophenylbis(triflyl)methane can be obtained at 95% yield with Tf₂O as a base. Consequently, the reactions of pentafluorophenylbis(triflyl)methane with the various types of alkyl lithium reagents were examined, in order to determine the generality and range of the nucleophilic substitution specific to the para position of pentafluorophenylbis(triflyl)methane. Table 3 shows the types of alkyl lithium reagents, the reaction conditions and she yield of the para position substituents of pentafluorophenylbis(triflyl)methane. The para position substituents of pentafluorophenylbis(triflyl)methane shown in Table 3 are obtained by washing the reaction product obtained by reacting pentafluorophenylbis(triflyl)methane with alkyl lithium reagent, with a hydrochloric solution. The "Bn" shown in Table 3 represents a benzyl group.

**Table 3**

| | | |
|---|---|---|
| 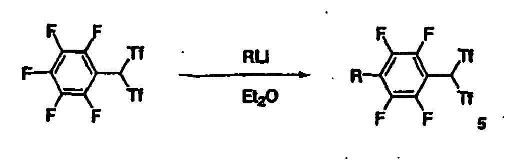 | | |

| RLi (equivalent weight) | Condition | 5, yield (%) |
|---|---|---|
| *t*-BuLi (3) | -78°C, 1 h | 87 |
| BuLi (3) | -78°C, 1 h | >95 |
| BnLi (5) | -78°C, 6 h | 83 |
| PhLi (3) | -78°C. to rt, 1 day | >95 . |
| 3,4,5-F₃C₆H₂Li (5) | -20°C to rt, 3 h | 75 |
| 3,5-(CF₃)₂C₆H₃Li (5) | -20°C to rt, 3 h | 70 |

### Example 6 [Synthesis of {4-(pentafluorophenyl)-2,3,5,6-tetrafluorophenyl}bis(triflyl)methane]

### Synthesis of 4-methylperfluorobiphenyl;

A diethylether solution of methyl lithium (13 mL, 15 mmol) was dropped for 0.5 hour to a THF (50 mL) solution dissolved with perfluorobiphenyl (10 g, 30 mmol), at -78°C under argon atmosphere. Then, after the solution was stirred at the same temperature for 2 hours, it was further stirred at room temperature for 2 hours. Water was added to stop the reaction, diethylether was used for extraction, and its organic phase was dried with magnesium sulfate. After filtration was conducted, the solvent was removed under reduced pressure, and a mixture of 4-methylperfluorobiphenyl, 4,4'-dimethylperfluorobiphenyl and perfluorobiphenyl (molar ratio, 30:3:67) was obtained as a crude product.

### Synthesis of 4-(bromomethyl)perfluorobiphenyl;

A mixed solution of a mixture comprising the 4-methylperfluorobiphenyl mentioned above, an N-bromo succinic imide (NBS) (26.7g, 150 mmol), AIBN (0.99 g, 6 mmol) and a carbon tetrachloride (100 mL) was subjected to heating under reflux for 1 week. During this process, the progress of the reaction was confirmed by TLC, and NBS and AIBN were added on a timely basis. Ultimately, 285 mmol NBS and 15 mmol AIBN were added to the solution. After the reaction was completed, the solution was cooled to room temperature, and the solvent was removed under reduced pressure. The crude product was purified by silica gel column chromatography (developing solvent: hexane-ethyl acetate = 100:1), and the 4-(bromomethyl)perfluorobiphenyl (7.36 g, 18 mmol, total yield from methyl lithium 60%) was isolated. ¹H NMR (CDCl₃, 300 MHz) δ4.58 (s, 2H, CH₂Br); ¹⁹F NMR (CDCl₃, 282 MHz) δ-138.02 (dd, J = 10.6, 19.8 Hz, 2F), -138.56 (dt, J = 9.1, 20.3 Hz, 2F), -142.36 (dd, J = 10.6, 19.8 Hz, 2F), -150.59 (t, J = 20.3 Hz, 1F), -161.08 (dt, J = 7.1, 20.3 Hz, 2F).

### Synthesis of {4-(pentafluorophenyl)-2,3,5,6-tetrafluorophenyl}(triflyl)methane;

4-bromomethylperfluorobiphenyl (3.68 g, 9 mmol) and sodium trifluoromethane sulfinate (1.69 g, 10.8 mmol) were dissolved in propionitrile (30 mL), and the resultant solution was subjected to heating under reflux for 12 hours. After the reaction, the solution was cooled to room temperature, and water was added for extraction with ethyl acetate. The organic phase was dried with magnesium sulfate, filtrated, and the solvent was removed under reduced pressure. The crude product was purified by silica gel column chromatography (hexane-ethyl acetate = 20:1 to 8:1 to 1:1), and the aimed {4-(pentafluorophenyl)-2,3,5,6-tetrafluorophenyl}(triflyl)methane (3.91 g, 8.46 mmol, 94% yield) was isolated.
¹H NMR (CDCl₃, 300 MHz) δ 4.75 (s, 2H, CH₂Tf); ¹⁹F NMR (CDCl₃, 282 MHz) δ-78.24 (s, 3F, CF₃), -136.82 to -136.62 (m, 1F), -137.72 (dd, J = 10.7, 18.3 Hz, 2F), -138.84 (dd, J = 10.7, 18.3 Hz, 2F), -149.69 (t, J = 21.3 Hz, 1F), -160.63 (dt, J = 6.1, 21.3 Hz, 2F).

### Synthesis of {4-(pentafluorophenyl)-2,3,5,6-tetrafluorophenyl}bis(triflyl)methane:

A tert-butyl magnesium chloride (5 mL, 10 mmol, 2.0 M diethylether solution) was added to a diethylether (120 mL) solution dissolved with {4-(pentafluorophenyl)-2,3,5,6-tetrafluorophenyl)(triflyl)methane (4.6 g, 10 mmol), at -78°C under argon atmosphere. After the reaction solution was stirred for 0.5 hour at -78°C, it was further stirred for 0.5 hour at 0°C. Then, the solution was cooled again to -78°C, trifluoromethane sulfonic acid anhydride (0.84 mL, 5 mmol) was added, and the resultant solution was stirred for 2 hours at room temperature. Further, tert-butyl magnesium chloride (3.75 mL, 7.5 mmol, 2.0 M diethylether solution) was added at -78°C. After the reaction solution was stirred at -78°C for 0.5 hour, it was stirred at 0° C for 0.5 hour. The solution was cooled again to -78° C, trifluoromethane sulfonic acid anhydride (0.84 mL, 5 mmol) was added, and the resultant solution was stirred for 2 hours at room temperature. After the reaction was completed, water was added, further neutralized with 1 M hydrochloric acid water, and the water phase was washed with hexane. Then, said water phase was acidified with 4 M hydrochloric acid water, and extracted with diethylether. The organic phase was dried with magnesium sulfate, filtrated, and the solvent was removed under reduced pressure. The crude product was sublimated (8 to 9 Pa, 150°C, and the aimed {4-(pentafluorophenyl) -2,3,5,6-tetrafluorophenyl}bis(triflyl)methane (2.79 g, 4.7 mmol, 47% yield) was isolated.
¹H NMR (CDCl₃, 300 MHz) δ 6.32 (s, 1H, CH), ¹⁹F NMR (CDCl₃, 282 MHz) δ -75.1 (s, 6F, 2CF₃), -127.72 to -127.58 (m, 1F), -133.43 (dt, J = 10.2, 21.3 Hz, 1F), -134.60 (dt, J = 9.4, 21.3 Hz, 1F), -137.08 to -137.35 (m, 2F), -140.07 (br, 1F), -148.38 (t, J = 21.3 Hz, 1F), -160.01 (dt, J = 6.2, 21.3 Hz, 2F).

### Example 7 [Synthesis of 4'- Position Alkyl Substituent of {4-(pentafluorophenyl)-2,3,5,6-tetrafluorophenyl}bis(triflyl)methane]

Phenyl lithium (0.28 mL, 0.3 mmol, 1.06 M cyclohexane-diethylether mixed solution) was added to a diethylether (1 mL) solution dissolved with (4-(pentafluorophenyl)-2,3,5,6-tetrafluorophenyl}bis(triflyl)methane (59 mg, 0.1 mmol), at -78° C. under argon atmosphere. The temperature of the reaction solution was slowly raised to -40°C. and the solution was stirred for 1 hour. Water was added to stop the reaction, the solution was neutralized with 1 M hydrochloric acid water, and the water phase was washed with hexane. Subsequently, the solution was acidified with 4 M hydrochloric acid water, and extracted with diethylether. The organic phase was dried with magnesium sulfate, filtrated, and the solvent was removed under reduced pressure. The product obtained thereby was the aimed compound, {4-(4-phenyl-2,3,5,6-tetrafluorophenyl)-2,3,5,6-tetrafluorophenyl}bis(triflyl)methane (57.2 mg, 0.088 mmol, 88% yield), and no further purification was required. ¹H NMR (CDCl₃, 300 MHz) δ 6.33 (s, 1H, CH), 7. 54 (s, 5H, C₆H₅); ¹⁹F NMR (CDCl₃, 282 MHz) δ-75.15 (s, 6F, 2CF₃), -128.03 (dt, J = 10.5, 21.2 Hz, 1F), -133.35 (dt, J = 10.5, 21.2 Hz, 1F), -134.52 (dt, J = 10.5, 21,2 Hz, 1F) , -138 . 73 to -138 . 53 (m, 2F) , -140.42 (br, 1F), -142.66 to -142.53 (m, 2F).

### Example 8 [Synthesis of 4' Position Alkoxy Substituent of {4-(pentafluorophenyl)-2,3,5,6-tetrafluorophenyl}bis(trifly 1)methane]

Hexanol (1.5 mL, 12 mmol) was added to a pyridine (10 mL) solution dissolved with 60% sodium hydride containing mineral oil (0.56 g, 14 mmol) at 0°C, and the resultant solution was stirred for 1 hour at room temperature. After the solution was cooled to -20°C, {4-(pentafluorophenyl)-2,3,5,6-tetrafluorophenyl}bis (triflyl)methane (1.2 g, 2 mmol) was added. The reaction solution was stirred at -20 °C for 5 hours. After the reaction, the solution was acidified with 4 M hydrochloric acid water, and was extracted with diethylether. After drying with magnesium sulfate, the solution was filtrated, and the solvent was removed under reduced pressure. The crude product was purified by sublimation (0.2 to 0.3 torr, 80°C), and {4-(4-hexyloxy-2,3,5,6-tetrafluorophenyl)-2,3,5,6-tetrafluo rophenyl}bis(triflyl)methane was obtained at 94% yield (1.27 g, 1.88 mmol).
¹H NMR (CDCl₃, 300 MHz) δ 0.83 to 0.99 (m, 3H), 1.26 (br, 2H), 1.33 to 1.38 (m, 2H), 1.45 to 1.52 (m, 2H), 1.84 (5, J = 6.8 Hz, 2H), 4.38 (t, J=6.8 Hz, 2H), 4.32 (s, 1H); ¹⁹F NMR (CDCl₃, 282 MHz) δ-75.0 (s, 6F, 2CF₃), -128. 4 to -128. 3 (m, 1F), -133. 6 (dt, J = 9.1, 21.3 Hz, 1F), -134.8 (dt, J = 9.1, 21. 3 Hz, 1F) -139.8 to -139.6 (m, 2F), -140.7 (br, 1F), -156.5 (d, J = 19.7 Hz, 2F).

### Example 9 [Synthesis of Para Position Alkoxy Substituent of Pentafluorophenylbis(triflyl)methane]

Hexanol (1.5 mL, 12 mmol) was added to a pyridine (10 mL) solution dissolved with 60% sodium hydride containing mineral oil (0.56 g, 14 mmol) at 0°C, and the resultant solution was stirred for 1 hour at room temperature. After the solution was cooled to -20°C, pentafluorophenylbis(triflyl)methane (0.89g, 2 mmol) was added. The reaction solution was stirred at -20°C for 5 hours. After the reaction, the solution was acidified with 4 M hydrochloric acid water, and was extracted with diethylether. After drying with magnesium sulfate, the solution was filtrated, and the solvent was removed under reduced pressure. The crude product was purified by sublimation (0.2 to 0.3 torr, 65°C), and 4-hexyloxy-2,3,5,6-tetrafluorophenylbis(triflyl)methane was obtained at 99% yield (1.02 g, 1.98 mmol).
¹H NMR (CDCl₃, 300 MHz) δ0.91 (t, J=7.1 Hz, 3H), 1.32 to 1.37 (m, 4H), 1.43 to 1.51 (m, 2H), 1.83 (quintet, J = 6.8 Hz, 2H), 4.44 (t, J = 6.8 Hz, 2H) , 6.19 (s, 1H) ; ¹⁹F NMR (CDCl₃, 282 MHz) δ-75.35 (s, 6F, 2CF₃), -130.64 (dt, J= 9.9, 21.2 Hz, 1F), -143.16 (br, 1F), -155.31 (d, J = 21.2 Hz, 1F).

### Example 10 [Synthesis of Lithium Pentafluorophenylbis(triflyl)methide]

The pentafluorophenylbis(triflyl)methane obtained from Example 4 (1 mmol) and LiOH·H₂O (1 mmol) were dissolved in a diethylether (10 mL), the resultant solution was stirred at room temperature for 12 hours , then concentrated and dried to obtain a white powder, lithium pentafluorophenylbis(triflyl)methide (100% yield). The physical property of this lithium pentafluorophenylbis(triflyl)methide obtained is as follows.

Lithium pentafluorophenylbis(triflyl)methide (Lithium Pentafluorophenylbis(triflyl)methide): ¹³C NMR (CD,OD, 125 MHz) δ 56.1, 109.0 (dt. J = 4, 19 Hz, 1C, ipso-C), 122.3 (q, J_{CF} = 324 Hz, 2C, 2CF₃) 138.5 (d, J_{CF} = 247 Hz, 2C, 2m-C). 143.0 (d, J_{CF} = 251 Hz, 1C, p-C), 149.5 (d, J_{CF} = 245 Hz, 2C. 2o-C).

### Example 11 [Synthesis of Lithium{4-(pentafluorophenyl)-2,3,5,6-tetrafluorophenyl}bis(triflyl)methide]

The {4-(pentafluorophenyl)-2,3,5,6-tetrafluorophenyl}bis(triflyl)methane obtained from Example 6 (1 mmol) and LiOH·H₂O (1 mmol) were dissolved in a diethylether (10 mL), the resultant solution was stirred at room temperature for 12 hours, then concentrated and dried to obtain a white solid, lithium{4-(pentafluorophenyl)-2,3,5.6-tetrafluorophenyl}bis(triflyl)methide (100% yield).

### Example 12 [Synthesis of Silver (I) Pentafluorophenylbis(triflyl)methide]

Ag₂CO₃ (66 mg, 0.24 mmol) was added to an aqueous solution (3 mL) of pentafluorophenylbis(triflyl)methane (0.20 g, 0.40 mmol) in a reaction flask wherein light was shut out by an aluminum foil. The solution was stirred at room temperature for 12 hours, then filtrated if there were any solid remaining, followed by concentration. A white solid of silver (I) pentafluorophenylbis(triflyl)methide was obtained thereby (99% yield or more). The physical property of this silver (I) pentafluorophenylbis(triflyl)methide obtained is as follows.

Silver (I) pentafluorophenylbis(triflyl)methide (Silver(I) Pentafluorophenylbis(triflyl)methide): ¹⁹F NMR (CDCl₃, 282 MHz) δ -162.6 (dt, J = 7.6, 21.4 Hz, 2F, 2m-F) , -150.6 (t, J=21.4 Hz, 1F, p-F), -134.7-134.6 (m, 2F, 2o-F), -79.5 (s, 6F, 2CF₃)

### Example 13 [Synthesis of Scandium (III) Pentafluorophenylbis(triflyl)methide (Part 1)]

Sc₂O₃ (21 mg, 0.155 mmol) and pentafluorophenylbis(triflyl)methane (0.277 g, 0.62 mmol) were subjected to heating under reflux in water (0.5 mL) for 12 hours. Then, the unreacted Sc₂O₃ was removed by filtration and condensation was conducted. The crude product obtained was washed with chloroform the unreacted pentafluorophenylbis(triflyl)methane was removed, pressure was reduced by a vacuum pump, and then dried at 100° C to obtain a white powder of scandium (III) pentafluorophenylbis(triflyl)methide (50% yield).

### Example 14 [Synthesis of Scandium (III) Pentafluorophenylbis(triflyl)methide (Part 2)]

The silver (I) pentafluorophenylbis(triflyl)methide obtained from Example 12 (0.19 g, 0.34 mmol) and Sc (III) Cl₃·(H₂O)₆ (29 mg, 0.11 mmol) were stirred in a diethylether (3 mL) at room temperature for 12 hours. Then, silver chloride was removed by filtration and condensation was conducted. The unreacted pentafluorophenylbis(triflyl)methane was removed, pressure was reduced by a vacuum pump, and then dried at 100°C to obtain a white powder of scandium (III) pentafluorophenylbis(triflyl)methide (50% yield). The physical property of the scandium (III) pentafluorophenylbis(triflyl)methide obtained from the present Example and Example 13 is as follows.

Scandium (III) pentafluorophenylbis(triflyl)methide (Scandium(III) Pentafluorophenylbis(triflyl)methide): Mp. > 250°C (decomposed) ; ¹³CNMR (CD₃OD (δ 49.0), 125 MHz) δ 56.2, 109.0 (dt, J_{CF} = 2, 20 Hz, 1C, ipso-C), 122.3 (q, J_{CF} = 324 Hz, 2C, 2CF₃) , 137.8 (d, J_{CF} = 247 Hz, 2C, 2m-C) , 142.3 (d, J_{CF} = 251 Hz, 1C, p-C), 148.9 (d, J_{CF} =.245 Hz, 2C, 2o-C) ; ¹⁹F NMR (CD₃OD, 282 MHz) δ -166.4 (dt, J = 6.1, 21.3 Hz, 2F, 2m-F), -155.9 (t, J = 21.3 Hz, 1F, p-F), -134.9 to -134.9 (m, 2F, 2o-F), -80.9 (s, 6F, 2CF₃).

### Example 15 [Benzoylation Reaction of Menthol]

Benzoylation reaction was conducted to a menthol wherein the scandium (III) pentafluorophenylbis(triflyl)methide obtained from Examples 13 and 14 was used as a Lewis acid catalyst (Chemical formula 38). 1-menthol (0.18 g, 1 mmol ) and benzoic anhydride (0.34 g, 1.5 mmol) were reacted while stirring in acetonitrile (4.8 mL) at 26°C for 1 day, under the presence of the scandium (III) pentafluorophenylbis(triflyl)methide as 1 mol% of Lewis acid catalyst. Two to 3 drops of triethylamine was added to stop the reaction, the solution was added with 5 mL water, then concentrated and dried, and the menthyl benzoate produced was extracted with diethyl ether. The organic phase was analyzed with ¹HNMR and it was found that the menthyl benzoate showed a high yield of 79%. The same reaction as mentioned above was conducted, except for the use of scandium (III) triflate [Sc(OTf)₃] as a conventionally known Lewis acid catalyst, instead of the scandium (III) pentafluorophenylbis(triflyl)methide mentioned above. The yield of menthyl benzoate was only 98%. Based on these results, it was found out that the catalytic activity of the scandium (III) pentafluorophenylbis(triflyl)methide of the present, invention is much more higher compared to that of the existing Lewis acid catalysts.

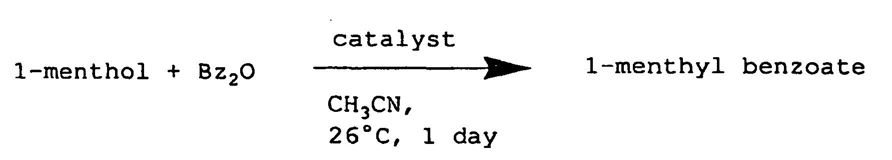

### Example 16 [Diels-Alder Reaction of Methacrolein]

Diels-Alder reaction was conducted to a methacrolein wherein the scandium (III) pentafluorophenylbis(triflyl)methideobtainedfrom Examples 13 and 14 was used as a Lewis acid catalyst (Chemical formula 39) Methacrolein (0.21 mL, 2.6mmol) and cyclopentadiene (0.56 mL, 6.8 mmol) were reacted while stirring in 3 mL dichloromethane at -40°C for 4 hours, under the presence of scandium (III) pentafluorophenylbis(triflyl)methide as 1 mol% of Lewis acid catalyst. Two to 3 drops of triethylamine was added to stop the reaction, 5 mL of water was added and the 5-norbornene-2-aldehyde produced was extracted with pentane. After concentration and drying, the crude product was analyzed with ¹HNMR and it was found that the 5-norbornene-2-aldehyde showed a high yield of 95% (88% exo). The same reaction as mentioned above was conducted, except for the use of scandium (III) triflate [Sc(OTf)₃] as a conventionally known Lewis acid catalyst, instead of the scandium (III) pentafluorophenylbis(triflyl)methide mentioned above. The 5-norbornene-2-aldehyde was hardly synthesized. Subsequently, 2 mol% of this scandium, (III) triflate was used, and the yield of the 5-norbornene-2-aldehyde was 97 % (89% exo). Based on these results, it was found out that the catalytic activity of the scandium (III) pentafluorophenylbis(triflyl)methide of the present invention is much more higher compared to that of the existing Lewis acid catalysts.

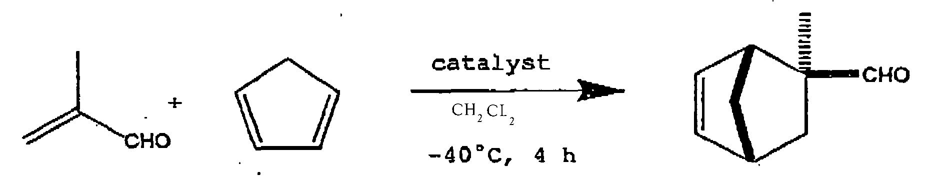

As can be seen from the Examples 5 and 9 and Examples 7 and 8 mentioned above, alkyl lithium generates a nucleophilic substitution reaction specifically to the para position of pentafluorophenylbis(triflyl)methane, whereas a nucleophilic substitution reaction is generated electively at the 4' position when reacted with {4-(pentafluorophenyl)-2,3,5,6-tetrafluorophenyl}bis(trifly 1)methane. Further, it was found out that as a nucleophilic reactant, not only limited to alkyl anion such as alkyl lithium and the like, alkoxy anion also shows the same reactivity. Moreover, as can be seen from the structural formula shown below and the pKa value (acetic acid) represented by the numeric value below the formula, when the para position of pentafluorophenylbis(triflyl)methane is substituted by an electron-donating group such as the alkyl group or the alkoxy group, its acidity decreases. However, when the same substituent was introduced to the 4' position of {4-(pentafluorophenyl)-2,3,5,6-tetrafluorophenyl}bis(trifly 1) methane, its acidity did not decrease. This fact reveals that the super strong acidity of {4-(pentafluorophenyl)-2,3,5,6-tetrafluorophenyl}bis(trifly 1)methane can be maintained even when it is supported on a resin or the like, with the use of the nucleophilic substitution reaction to the 4' position of {4-(pentafluorophenyl)-2,3,5,6-tetrafluorophenyl}bis(trifly 1)methane. It shows that the {4-(pentafluorophenyl)-2,3,5,6-tetrafluorophenyl}bis(trifly 1)methane is very useful as a synthesis material for various organic materials and acid catalysts with the use of super strong acidity.

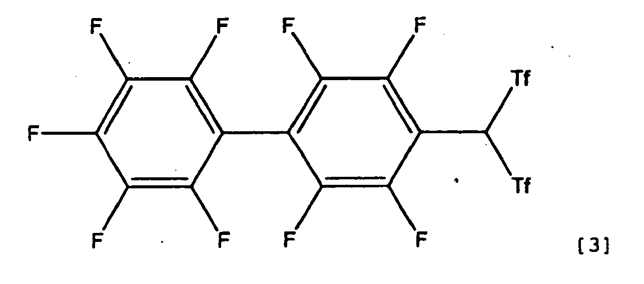

### Industrial Applicability

An arylbis(triflyl)methane having strong acidity can be produced efficiently and easily by the method for production according to the present invention. Since the pentafluorophenylbis(triflyl)methane and the like of the present invention are organic acids that are stronger than TfOH, their use as a novel type of conjugate base of protonic acid or metallic salt is expected. Moreover, since various types of aryl group can be introduced for an aryl group of arylbis(triflyl)methane, a wide application to asymmetric catalyst, functional material and the like is possible.

Further, the metallic arylbis(perfluoroalkylsulfonyl)methide such as scandium (III) pentafluorophenylbis(triflyl)methide and the like of the present invention shows a much better catalytic activity compared to that of the existing Lewis acid catalysts, and an organic compound can be easily synthesized at a high yield. The aryl group of metallic arylbis(perfluoroalkylsulfonyl)methide of the present invention can be introduced with various types of aryl group, and therefore, a wide application to asymmetric catalyst, functional material and the like is expected.

## Claims

1. An arylbis(perfluoroalkylsulfonyl)methane represented by the following general formula [1]: 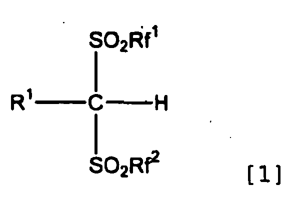 wherein R¹ is a 2,4,6-trimethylphenyl group, a 4-(trifluoromethyl)phenyl group, a 3,5-bis(trifluoromethyl)-phenyl group, a pentafluorophenyl group or a para substituted derivative thereof or a perfluorobiphenyl group or a 4¹-substituted derivative thereof, and Rf¹ and Rf² are independent to each other and represent a perfluoroalkyl group.

2. The arylbis(perfluoroalkylsulfonyl)methane according to claim 1 wherein Rf¹ and Rf² are both a trifluoromethyl group.

3. A pentafluorophenylbis(trifluoromethylsulfonyl)methane according to claim 1 represented by formula [2]: 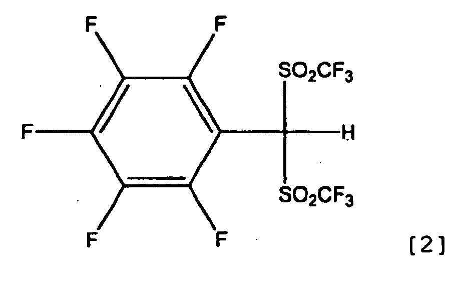 or a para substituted derivative thereof.

4. A {4-(pentafluorophenyl)-2,3,5,6-tetrafluorophenyl}-bis(triflyl)methane according to claim 1 represented by formula [3]: 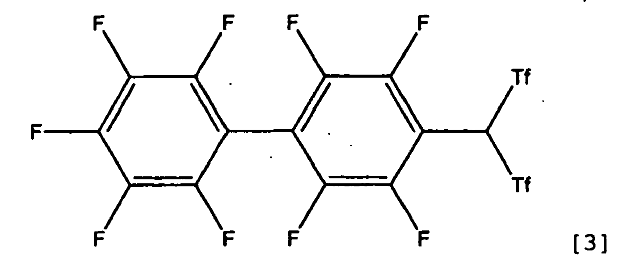 or a 4'-substituted derivative thereof.

5. A method for producing an arylbis(perfluoroalkylsulfonyl)methane as claimed in claim 1 wherein an aryl halomethane is reacted with a perfluoroalkyl sulfinate, the arylmethylperfluoroalkylsulfone produced is reacted with a deprotonation agent comprising an organic metal or a metallic salt, and the metallic salt of the arylmethylperfluoroalkylsulfone obtained is reacted with a perfluoroalkyl sulfonic acid anhydride.

6. The method according to claim 5, wherein the aryl halomethane is reacted with the perfluoroalkyl sulfinate by heating under reflux using a solvent with or without the presence of a catalyst.

7. The method according to claim 6, wherein tetrabutyl ammonium iodide is used as the catalyst.

8. The method according to claim 6, wherein propionitrile is used as the solvent.

9. The method according to claim 5, wherein the deprotonation agent comprising" 1.7 to 2.4 equivalent weight of an organic metal or a metallic salt is reacted with the arylmethylperfluoroalkylsulfone.

10. The method according to claim 5, wherein the aryl halomethane is 2,4,6-trimethylphenylmethyl chloride, 4-(trifluoromethyl)phenylmethyl bromide, 3,5-bis(trifluoromethyl)phenylmethyl bromide, pentafluorophenylmethyl bromide, or 4-(bromomethyl)perfluorobiphenyl.

11. The method according to claim 5, wherein the perfluoroalkyl sulfinate is an alkali metallic salt of trifluoromethane sulfinic acid.

12. The method according to claim 5, wherein the metallic salt of the arylmethylperfluoroalkylsulfone is a lithium salt or a magnesium salt of the arylmethylperfluoroalkylsulfone.

13. A metallic salt of arylbis(perfluoroalkyl-sulfonyl)-methane represented by general formula [6]: 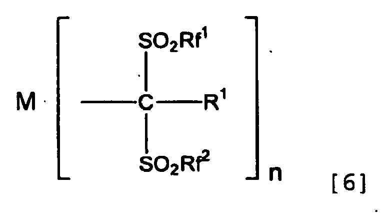 wherein R¹, Rf¹ and Rf² are as defined in claim 1, M represents any one of the elements selected from an alkali metallic element, an alkaline earth metallic element, a transition metallic element, boron, silicon, aluminum, tin, zinc or bismuth, and n represents a numeric value equal to the valence of M.

14. The metallic salt of arylbis(perfluoroalkylsulfonyl) methane according to claim 13, wherein the transition metallic element is any one of the metallic elements selected from scandium, yttrium, lanthanoid, copper, silver, titanium, zirconium or hafnium.

15. The metallic salt of arylbis(perfluoroalkylsulfonyl)methane according to claim 13, wherein the metallic salt of arylbis(perfluoroalkylsulfonyl)methane is a metallic salt of 2,4,6-trimethylphenylbis(triflyl)-methane, a metallic salt of 4-(trifluoromethyl)phenylbis-(triflyl)methane, a metallic salt of 3,5-bis(trifluoromethyl)phenylbis(triflyl)methane, a metallic salt of pentafluorophenylbis(triflyl)methane or a metallic salt of (4-(pentafluorophenyl)-2,3,5,6-tetra-fluorophenyl)bis-(triflyl)methane.

16. The metallic salt of arylbis(perfluoroalkylsulfonyl)methane according to claim 13, wherein the metallic salt of pentafluorophenylbis(triflyl)methane is a scandium (III) pentafluorophenylbis(triflyl)methide or a lithium pentafluorophenylbis(triflyl)methide.

17. The metallic salt of arylbis(perfluoroalkylsulfonyl)-methane according to claim 13, wherein the metallic salt of {4-(pentafluorophenyl)-2,3,5,6-tetra-fluorophenyl}bis-(triflyl)methane is a scandium (III) {4-(pentafluorophenyl)-2,3,5,6-tetrafluorophenyl}bis(triflyl)methide or a lithium {4-(pentafluorophenyl)-2,3,5,6-tetrafluorophenyl}bis(triflyl)methide.

18. A method for producing a metallic salt of arylbis-(perfluoroalkylsulfonyl)methane as claimed in claim 13 wherein an arylbis(perfluoroalkylsulfonyl)methane as claimed in claim 1 is neutralized with a hydroxide of a metal.

19. The method according to claim 18, wherein the hydroxide of the metal is a hydroxide of a metal selected from an alkali metal or an alkaline earth metal.

20. A method for producing a metallic salt of arylbis-(perfluoroalkylsulfonyl)methane represented by general formula [6']: 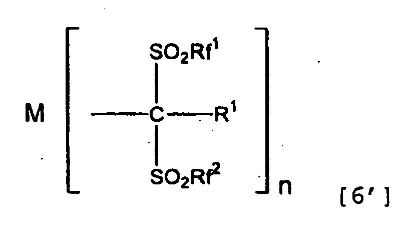 wherein R¹, Rf¹ and Rf² are as defined in claim 1, M represents a transition metallic element, and n represents a numeric value equal to the valence of M; wherein an arylbis(perfluoroalkylsulfonyl)methane as claimed in claim 1 is reacted with a salt or an oxide of transition metal by heating under reflux.

21. The method for producing the metallic salt of arylbis(perfluoroalkylsulfonyl)methane according to claim 20, wherein the salt or the oxide of transition metal is a lanthanoid metallic salt or a scandium oxide.

22. A method for producing a metallic salt as claimed in claim 13 wherein a metallic salt of an arylbis-(perfluoroalkylsulfonyl)methane as claimed in claim 1 is reacted with a halide of a metal having different metal species.

23. The method according to claim 22, wherein the metallic salt of arylbis(perfluoroalkylsulfonyl)methane as claimed in claim 13 is a silver salt of arylbis-(perfluoroalkylsulfonyl)methane.

24. A method for producing a silver salt of an arylbis-(perfluoroalkylsulfonyl)methane represented by general formula [6"]: 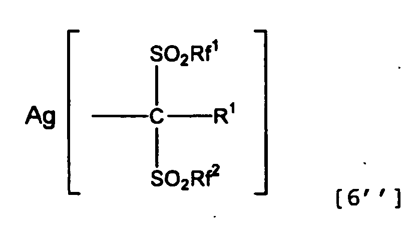 wherein R¹, Rf¹ and Rf² are as defined in claim 1, wherein an arylbis(perfluoroalkylsulfonyl)methane as claimed in any one of claims 1 to 4 is reacted with silver carbonate under shade.

25. The method for producing the metallic salt of arylbis(perfluoroalkylsulfonyl)methane according to any one of claims 18, 20, 22 or 24, wherein the arylbis-(perfluoroalkylsulfonyl)methane is obtained by reacting an aryl halomethane with a perfluoroalkyl sulfinate, followed by reacting the arylmethylperfluoroalkylsulfone produced with a deprotonation agent comprising an organic metal or a metallic salt, and the metallic salt of the arylmethylperfluoroalkylsulfone obtained is reacted with a perfluoroalkyl sulfonic acid anhydride.

26. The method for producing the metallic salt of arylbis(perfluoroalkylsulfonyl)methane according to claim 25, wherein the aryl halomethane is reacted with the perfluoroalkyl sulfinate by heating under reflux using a solvent with or without the presence of a catalyst.

27. The method for producing the metallic salt of arylbis(perfluoroalkylsulfonyl)methane according to claim 26, wherein tetrabutyl ammonium iodide is used as the catalyst.

28. The method for producing the metallic salt of arylbis(perfluoroalkylsulfonyl)methane according to claim 27, wherein propionitrile is used as the solvent.

29. The method for producing the metallic salt of arylbis(perfluoroalkylsulfonyl)methane according to claim 28, wherein the perfluoroalkyl sulfinate is an alkali metallic salt of trifluoromethane sulfinic acid.

30. The method for producing the metallic salt of arylbis(perfluoroalkylsulfonyl)methane according to claim 26, wherein the metallic salt of arylmethylperfluoroalkylsulfone is a lithium salt or a magnesium salt of arylmethylperfluoroalkylsulfone.

31. A catalyst comprising a metallic salt of the arylbis-(perfluoroalkylsulfonyl)methane represented by general formula [6] as claimed in any one of claims 13 to 17 as an active ingredient.

32. Use of the catalyst according to claim 31 as a Lewis acid catalyst.

33. A method for synthesizing an organic compound by using a catalyst according to claim 31 or 32, wherein the catalytic reaction is a benzoylation reaction or a Diels-Alder reaction and is conducted under the presence of said catalyst in a solvent.

## Patentansprüche

1. Arylbis-(perfluoralkylsulfonyl)-methan, repräsentiert durch die folgende allgemeine Formel [1]: 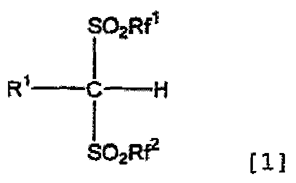 wobei R¹ eine 2,4,6-Trimethylphenyl-Gruppe, eine 4-(Trifluormethyl)-phenyl-Gruppe, eine 3,5-Bis-(trifluormethyl)-phenyl-Gruppe, eine Pentafluorphenyl-Gruppe oder ein para-substituiertes Derivat davon oder eine Perfluorbiphenyl-Gruppe oder ein 4'-substituiertes Derivat davon ist und Rf¹ und Rf² voneinander unabhängig sind und eine Perfluoralkyl-Gruppe repräsentieren.

2. Arylbis-(perfluoralkylsulfonyl)-methan nach Anspruch 1, wobei Rf¹ und Rf² beide eine Trifluormethyl-Gruppe sind.

3. Pentafluorphenylbis-(trifluormethylsulfanyl)-methan nach Anspruch 1, repräsentiert durch Formel [2]:

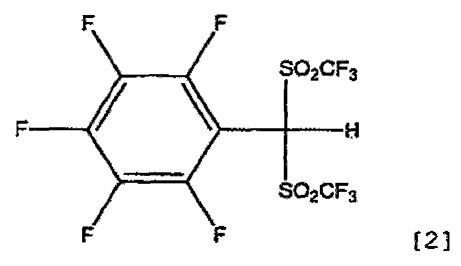

4. {4-(Pentafluorphenyl)-2,3,5,6-tetrafluorphenyl}-bis-(triflyl)-methan nach Anspruch 1, repräsentiert durch Formel [3]: 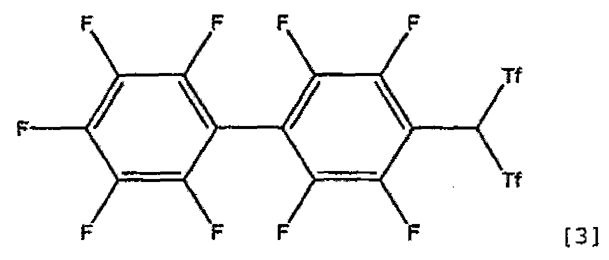 oder ein 4'-substituiertes Derivat davon.

5. Verfahren zum Herstellen eines Arylbis-(perfluoralkylsulfonyl)-methans nach Anspruch 1, wobei ein Arylmethylhalogenid mit einem Perfluoralkylsulfinat umgesetzt wird, das erzeugte Arylmethylperfluoralkylsulfon mit einem Deprotonierungsmittel umgesetzt wird, welches ein organisches Metall oder ein Metallsalz umfaßt, und das Metallsalz des erhaltenen Arylmethylperfluoralkylsulfons mit einem Perfluoralkylsulfonsäureanhydrid umgesetzt wird.

6. Verfahren nach Anspruch 5, wobei das Arylmethylhalogenid mit dem Perfluoralkylsulfinat durch Erhitzen unter Rückfluß unter Verwendung eines Lösungsmittels mit oder ohne Gegenwart eines Katalysators umgesetzt wird.

7. Verfahren nach Anspruch 6, wobei Tetrabutylammoniumiodid als Katalysator verwendet wird.

8. Verfahren nach Anspruch 6, wobei Propionitril als Lösungsmittel verwendet wird.

9. Verfahren nach Anspruch 5, wobei das Deprotonierungsmittel, welches 1,7 bis 2,4 Gewichtsäquivalente eines organischen Metalls oder eines Metallsalzes umfaßt, mit dem Arylmethylperfluoralkylsulfon umgesetzt wird.

10. Verfahren nach Anspruch 5, wobei das Arylmethylhalogenid 2,4,6-Trimethylphenylmethylchlorid, 4-(Trifluormethyl)-phenylmethylbromid, 3,5-Bis-(trifluormethyl)-phenylmethylbromid, Pentafluorphenylmethylbromid oder 4-(Brommethyl)-perfluorbiphenyl ist.

11. Verfahren nach Anspruch 5, wobei das Perfluoralkylsulfinat ein Alkalimetallsalz von Trifluormethansulfinsäure ist.

12. Verfahren nach Anspruch 5, wobei das Metallsalz des Arylmethylperfluoralkylsulfons ein Lithiumsalz oder ein Magnesiumsalz des Arylmethylperfluoralkylsulfons ist.

13. Metallsalz von Arylbis-(perfluoralkylsulfonyl).methan, repräsentiert durch die allgemeine Formel [6]: 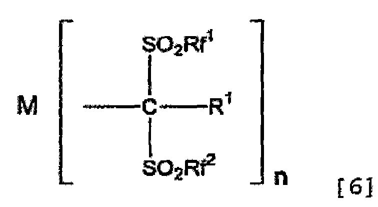 wobei R¹, Rf¹ und Rf² wie in Anspruch 1 definiert sind, M irgendeines der Elemente repräsentiert, die unter einem Alkalimetallelement, einem Erdalkalimetallelement, einem Übergangsmotallelement, Bor, Silicium. Aluminium, Zinn, Zink oder Wismut ausgewählt sind, und n einen numerischen Wert gleich der Wertigkeit von M repräsentiert.

14. Metallsalz von Arylbis-(perfluoralkylsulfonyl)-mefhan nach Anspruch 13, wobei das Übergangsmetallelement irgendeines der Metallelemente ist, ausgewählt unter Scandium, Yttrium, Lanthanoid, Kupfer, Silber, Titan, Zirkonium oder Hafnium.

15. Metallsalz von Arylbis-(pertluomlkylsulfonyl)-methan nach Anspruch 13, wobei das Metallsalz von Arylbis-(perfluoralkylsulfonyl)-methan ein Metallsalz von 2,4,6-Trimethylphenylbis-(triflyl)-methan, ein Metallsalz von 4-(Trifluormethyl)-phenylbis-(triflyl)-methan, ein Metallsalz von 3,5-Bis-(trifluormethyl)-phenylbis-(triflyl)-methan, ein Metallsalz von Pentafluorphenylbis-(triflyl)-methan oder ein Metallsalz von {4-(Pentafluorphenyl)-2,3,6,6,tetrafluorphenyl}-bis-(triflyl)-methan ist.

16. Metallsalz von Arylbis-(perfluoralkylsulfonyl)-methan nach Anspruch 13, wobei das Metallsalz von Pentafluorphenylbis-(triflyl)-methan ein Scandium(III)-pentafluorphenylbis-(triflyl)-methid oder ein Lithiumpentafluorphenylbis-(triflyl)-methid ist.

17. Metallsalz von Arylbis-(perfluoralkylsulfonyl)-methan nach Anspruch 13, wobei das Metallsalz von {4-(Pentafluorphenyl)-2,3,5,6-tetrafluorphenyl}-bis-(triflyl)-methan ein Scandium(III)-{4-(pentafluorphenyl)-2,3,5,6-tetrafluorphenyl}-bis-(triflyl)-methid oder ein Lithium-{4-(pentafluorphenyl)-2,3,5,6-tetrafluorphenyl}-bis-(triflyl)-methid ist.

18. Verfahren zum Herstellen eines Metallsalzes von Arylbis-(perfluoralkylsulfonyl)-methan nach Anspruch 13, wobei ein Arylbis-(perfluoralkylsulfonyl)-methan nach Anspruch 1 mit einem Hydroxid eines Metalls neutralisiert wird.

19. Verfahren nach Anspruch 18, wobei das Hydroxid des Metalls ein Hydroxid eines Metalls, ausgewählt unter einem Alkalimetall oder einem Erdalkalimetall, ist.

20. Verfahren zum Herstellen eines Metallsalzes von Arylbis-(perfluoralkylsulfonyl)-methan, repräsentiert durch die allgemeine Formel [6']: 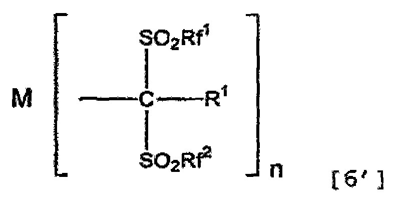 wobei R¹, Rf¹ und Rf² wie in Anspruch 1 definiert sind, M ein Übergangsmetallelement repräsentiert und n einen numerischen Wert gleich der Wertigkeit von M repräsentiert, wobei ein Arylbis-(perfluoralkylsulfonyl)-methan nach Anspruch 1 durch Erhitzen unter Rückfluß mit einem Salz oder einem Oxid des Übergangsmetalls umgesetzt wird.

21. Verfahren zum Herstellen des Metallsalzes von Arylbis-(perfluoralkylsulfonyl)-methan nach Anspruch 20, wobei das Salz oder das Oxid des Übergangsmetalls ein Lanthanoidmetallsalz oder ein Scandiumoxid ist.

22. Verfahren zum Herstellen eines Metallsalzes nach Anspruch 13, wobei ein Metallsalz eines Arylbis-(perfluoralkylsulfonyl)-methans nach Anspruch 1 mit einem Halogenid eines Metalls mit unterschiedlichen Metallspezies umgesetzt wird.

23. Verfahren nach Anspruch 22, wobei das Metallsalz von Arylbis-(perfluoralkylsulfonyl)-methan nach Anspruch 13 ein Silbersalz von Arylbis-(perfluoralkylsulfonyl)-methan ist.

24. Verfahren zum Herstellen eines Silbersalzes eines Arylbis-(perfluoralkylsulfonyl)-methans, repräsentiert durch die allgemeine Formel [6"]: 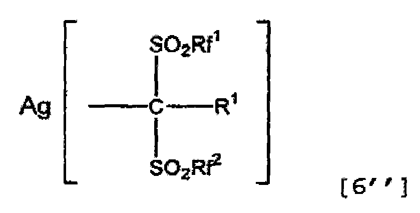 wobei R¹, Rf¹ und Rf² wie in Anspruch 1 definiert sind, wobei ein Arylbis-(perfluoralkylsulfonyl)-methan nach einem der Ansprüche 1 bis 4 mit Silbercarbonat lichtgeschützt umgesetzt wird.

25. Verfahren zum Herstellen des Metallsalzes von Arylbis-(parfluoralkylsulfonyl)-methan nach einem der Ansprüche 18, 20, 22 oder 24, wobei das Arylbis-(perfluoralkylsulfonyl)-methan erhalten wird durch Umsetzen eines Arylmethylhalogenids mit einem Perfluoralkylsulfinat, gefolgt von Umsetzen des erzeugten Arylmethylperfluoralkylsulfons mit einem Deprotonierungsmittel, welches ein organisches Metall oder ein Metallsalz umfaßt, wobei das erhaltene Metallsalz des Arylmethylperfluoralkylsulfons mit einem Perfluoralkylsulfonsäureanhydrid umgesetzt wird.

26. Verfahren zum Herstellen des Metallsalzes von Arylbis-(perfluoralkylsulfonyl)-methan nach Anspruch 25, wobei das Arylmethylhalogenid mit dem Perfluoralkylsulfinat durch Erhitzen unter Rückfluß unter Verwendung eines Lösungsmittels mit oder ohne Gegenwart eines Katalysators umgesetzt wird.

27. Verfahren zum Herstellen des Metallsalzes von Arylbis-(perfluoralkylsulfonyl)-methan nach Anspruch 26, wobei Tetrabutylammoniumiodid als Katalysator verwendet wird.

28. Verfahren zum Herstellen des Metallsalzes von Arylbis-(perfluoralkylsulfonyl)-methan nach Anspruch 27, wobei Propionitril als Lösungsmittel verwendet wird.

29. Verfahren zum Herstellen des Metallsalzes von Arylbis-(perfluoralkylsulfonyl)-methan nach Anspruch 28, wobei das Perfluoralkylsulfinat ein Alkalimetallsalz von Trifluormethansulfinsäure ist.

30. Verfahren zum Herstellen des Metallsalzes von Arylbis-(perfluoralkylsulfonyl)-methan nach Anspruch 26, wobei das Metallsalz von Arylmethylperfluoralkylsulfon ein Lithiumsalz oder ein Magnesiumsalz von Arylmethylperfluoralkylsulfon ist.

31. Katalysator, welcher ein Metallsalz des Arylbis-(perfluoralkylsulfonyl)-methans, repräsentiert durch die allgemeine Formel [6] nach einem der Ansprüche 13 bis 17 als einen aktiven Inhaltsstoff umfaßt.

32. Verwendung des Katalysators nach Anspruch 31 als Lewis-Säure-Katalysator.

33. Verfahren zum Synthetisieren einer organischen Verbindung unter Verwendung eines Katalysators nach Anspruch 31 oder 32, wobei die katalytische Reaktion eine Benzoylierungsreaktion oder eine Diels-Alder-Reaktion ist und in Gegenwart des Katalysators in einem Lösungsmittel durchgeführt wird.

## Revendications

1. Arylbis(perfluoroalkylsulfonyl)méthane représenté par la formule générale [1] suivante : 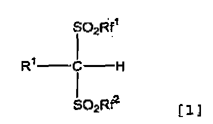 où R¹ est un groupe 2,4,6-triméthylphényle, un groupe 4-(trifluorométhyl)phényle, un groupe 3,5-bis(trifluorométhyl)phényle, un groupe pentafluorophényle ou un dérivé para-substitué de celui-ci ou un groupe perfluorobiphényle ou un dérivé 4'-substitué de celui-ci, et Rf¹ et Rf² sont indépendants l'un de l'autre et représentent un groupe perfluoroalkyle.

2. Arylbis(perfluoroalkylsulfonyl)méthane selon la revendication 1 où Rf¹ et Rf² sont l'un et l'autre un groupe trifluorométhyle.

3. Pentafluorophénylbis(trifluorométhylsulfonyl)méthane selon la revendication 1 représenté par la formule [2] : 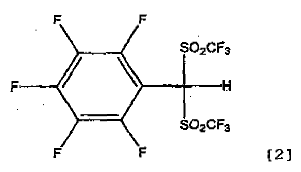 ou dérivé para-substitué de celui-ci.

4. {4-(pentafluorophényl)-2,3,5,6-tétrafluorophényl}-bis(triflyl)-méthane selon la revendication 1 représenté par la formule [3] : 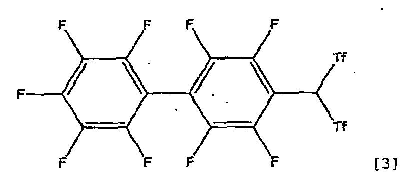 ou dérivé 4'-substitué de celui-ci.

5. Procédé pour produire un arylbis(perfluoroalkylsulfonyl)méthane selon la revendication 1 où un arylhalogénométhane est mis à réagir avec un sulfinate de perfluoroalkyle, l'arylméthylperfluoroalkylsulfone produite est mise à réagir avec un agent de déprotonation comprenant un métal organique ou un sel métallique, et le sel métallique de l'arylméthylperfluoroalkylsulfone obtenu est mis à réagir avec un anhydride d'acide perfluoroalkylsulfonique.

6. Procédé selon la revendication 5 où l'arylhalogénométhane est mis à réagir avec le sulfinate de perfluoroalkyle par chauffage à reflux avec un solvant avec ou sans la présence d'un catalyseur.

7. Procédé selon la revendication 6 où l'iodure de tétrabutylammonium est utilisé comme catalyseur.

8. Procédé selon la revendication 6 où le propionitrile est utilisé comme solvant.

9. Procédé selon la revendication 5 où l'agent de déprotonation comprenant 1,7 à 2,4 poids équivalent d'un métal organique ou d'un sel métallique est mis à réagir avec l'arylméthylperfluoroalkylsulfone.

10. Procédé selon la revendication 5 où l'arylhalogénométhane est le chlorure de 2,4,6-triméthylphénylméthyle, le bromure de 4-(trifluorométhyl)-phénylméthyle, le bromure de 3,5-bis(trifluorométhyl)phénylméthyle, le bromure de pentafluorophénylméthyle ou le 4-(bromométhyl)perfluorobiphényle.

11. Procédé selon la revendication 5 où le sulfinate de perfluoroalkyle est un sel de métal alcalin de l'acide trifluorométhanesulfinique.

12. Procédé selon la revendication 5 où le sel métallique de l'arylméthylpexfluoroalkylsulfone est un sel de lithium ou un sel de magnésium de l'arylméthylperfluoroalkylsulfone.

13. Sel métallique d'arylbis(perfluoroalkylsulfonyl)méthane représenté par la formule générale [6] : 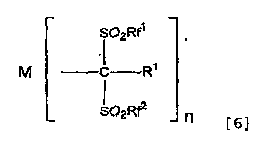 où R¹, Rf¹ et Rf² sont définis comme dans la revendication 1, M représente l'un quelconque des éléments choisis parmi un élément métallique alcalin, un élément métallique alcalino-terreux, un élément métallique de transition, le bore, le silicium, l'aluminium, l'étain, le zinc et le bismuth, et n représente une valeur numérique égale à la valence de M.

14. Sel métallique d'arylbis(perfluoroalkylsulfonyl)méthane selon la revendication 13 où l'élément métallique de transition est l'un quelconque des éléments métalliques choisis parmi le scandium, l'yttrium, un lanthanoïde, le cuivre, l'argent, le titane, le zirconium et le hafnium.

15. Sel métallique d'arylbis(perfluoroalkylsulfonyl)méthane selon la revendication 13 où le sel métallique d'arylbis(perfluoroalkylsulfonyl)méthane est un sel métallique de 2,4,6-triméthylphénylbis(triflyl)méthane, un sel métallique de 4-(trifluorométhyl)phénylbis(triflyl)méthane, un sel métallique de 3,5-bis(trifluorométhyl)phénylbis(triftyl)méthane, un sel métallique de pentafluorophénylbis(triflyl)méthane ou un sel métallique de {4-(pentafluorophényl)-2,3,5,6-tétra-fluorophényl}bis(triflyl)méthane.

16. Sel métallique d'arylbis(pertluoroalkylsulfonyl)méthane selon la revendication 13 où le sel métallique de pentafluorophénylbis(triflyl)méthane est un pentafluorophénylbis(triflyl)méthylure de scandium (III) ou un pentafluorophénylbis(triflyl)méthylure de lithium.

17. Sel métallique d'arylbis(perfluoroalkylsulfonyl)méthane selon la revendication 13 où le sel métallique de {4-(pentafluorophényl)-2,3,5,6-tétrafluorophényl}bis(triflyl)méthane est un {4-(pentafluorophényl)-2,3,5,6-tétrafluorophényl}bis(triflyl)méthylure de scandium (III) ou un {4-(pentafluorophényl)-2,3,5,6-tétrafluorophényl}bis(triflyl)méthylure de lithium.

18. Procédé pour produire un sel métallique d'arylbis(perfluoroalkylsulfonyl)méthane selon la revendication 13 où un arylbis(perfluoroalkylsulfonyl)méthane selon la revendication 1 est neutralisé avec un hydroxyde d'un métal.

19. Procédé selon la revendication 18 où l'hydroxyde du métal est un hydroxyde d'un métal choisi parmi un métal alcalin et un métal alcalino-terreux.

20. Procédé pour produire un sel métallique d'arylbis(perfluoroalkylsulfonyl)méthane représenté par la formule générale [6'] : 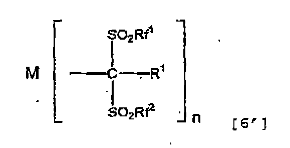 où R¹, Rf¹ et Rf² sont définis comme dans la revendication 1, M représente un élément métallique de transition et n représente une valeur numérique égale à la valence de M ;
où un arylbis(perfluoroalkylsulfonyl)méthane selon la revendication 1 est mis à réagir avec un sel ou un oxyde de métal de transition par chauffage à reflux.

21. Procédé pour produire le sel métallique d'arylbis(perfluoroalkylsulfonyl)méthane selon la revendication 20 où le sel ou l'oxyde de métal de transition est un sel métallique lanthanoïde ou un oxyde de scandium.

22. Procédé pour produire un sel métallique selon la revendication 13 ou un sel métallique d'un arylbis(perfluoroalkylsulfonyl)méthane selon la revendication 1 est mis à réagir avec un halogénure d'un métal ayant une espèce métallique différente.

23. Procédé selon la revendication 22 où le sel métallique d'arylbis-(perfluoroalkylsulfonyl)méthane selon la revendication 13 est un sel d'argent d'arylbis(perfluoroalkylsulfonyl)méthane.

24. Procédé pour produire un sel d'argent d'un arylbis(perfluoroalkylsulfonyl)méthane représenté par la formule générale [6"] : 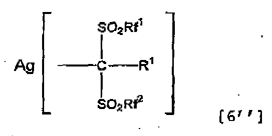 où R¹, Rf¹ et Rf² sont définis comme dans la revendication 1, où un arylbis(perfluoroalkylsulfonyl)méthane selon l'une quelconque des revendications 1 à 4 est mis à réagir avec le carbonate d'argent à l'abri de la lumière.

25. Procédé pour produire le sel métallique d'arylbis-(perfluoroalkylsulfonyl)méthane selon l'une quelconque des revendications 18, 20, 22 et 24 où l'arylbis(perfluoroalkylsulfonyl)méthane est obtenu par réaction d'un arylhalogénométhane avec un sulfinate de perfluoroalkyle, puis l'arylméthylperfluoroalkylsulfone produite est mise à réagir avec un agent de déprotonation comprenant un métal organique ou un sel métallique, et le sel métallique de l'arylméthylperfluoroalkylsulfone obtenu est mis à réagir avec un anhydride d'acide perfluoroalkylsulfonique.

26. Procédé pour produire le sel métallique d'arylbis(perfluoroalkylsulfonyl)méthane selon la revendication 25 où l'arylhalogénométhane est mis à réagir avec le sulfinate de perfluoroalkyl par chauffage à reflux avec un solvant avec ou sans la présence d'un catalyseur.

27. Procédé pour produire le sel métallique d'arylbis(perfluoroalkylsulfonyl)méthane selon la revendication 26 où l'iodure de tétrabutylammonium est utilisé comme catalyseur.

28. Procédé pour produire le sel métallique d'arylbis(perfluoroalkylsulfonyl)uréthane selon la revendication 27 où le propionitrile est utilisé comme solvant.

29. Procédé pour produire le sel métallique d'arylbis(perfluoroalkylsulfonyl)méthane selon la revendication 28 où le sulfinate de perfluoroalkyle est un sel métallique alcalin de l'acide trifluorométhane-sulfinique.

30. Procédé pour produire le sel métallique d'arylbis(perfluoroalkylsulfonyl)méthane selon la revendication 26 où le sel métallique d'arylméthylperfluoroalkylsulfone est un sel de lithium ou un sel de magnésium d'arylméthylperfluoroalkylsulfone.

31. Catalyseur comprenant un sel métallique de l'arylbis(perfluoroalkylsulfonyl)méthane représenté par la formule générale [6] selon l'une quelconque des revendications 13 à 17 comme ingrédient actif.

32. Utilisation du catalyseur selon la revendication 31 comme catalyseur de type acide de Lewis.

33. Procédé pour synthétiser un composé organique au moyen d'un catalyseur selon la revendication 31 ou 32 où la réaction catalytique est une réaction de benzoylation ou une réaction de Diels-Alder et est conduite en présence dudit catalyseur dans un solvant.
